(19) Europäisches Patentamt / European Patent Office / Office européen des brevets

(11) EP 4 467 543 A1

(12) EUROPEAN PATENT APPLICATION
published in accordance with Art. 153(4) EPC

(43) Date of publication:
**27.11.2024 Bulletin 2024/48**

(21) Application number: **23742991.5**

(22) Date of filing: **20.01.2023**

(51) International Patent Classification (IPC):
***C07D 471/04*** *(2006.01)* ***A61K 31/519*** *(2006.01)*
***A61K 31/517*** *(2006.01)* ***A61P 11/08*** *(2006.01)*

(52) Cooperative Patent Classification (CPC):
**A61K 31/517; A61K 31/519; A61P 11/08; C07D 471/04**

(86) International application number:
**PCT/CN2023/073288**

(87) International publication number:
**WO 2023/138676 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.01.2022 CN 202210070971**
**28.01.2022 CN 202210103923**

(71) Applicant: **Xizang Haisco Pharmaceutical Co., Ltd.**
**Lhoka, Tibet 856099 (CN)**

(72) Inventors:
- **LI, Yao**
  **Chengdu, Sichuan 611130 (CN)**
- **ZHANG, Guobiao**
  **Chengdu, Sichuan 611130 (CN)**
- **ZHANG, Xiaobo**
  **Chengdu, Sichuan 611130 (CN)**
- **ZHANG, Yaming**
  **Chengdu, Sichuan 611130 (CN)**
- **HUANG, Shilin**
  **Chengdu, Sichuan 611130 (CN)**
- **YAN, Linjie**
  **Chengdu, Sichuan 611130 (CN)**
- **TANG, Pingming**
  **Chengdu, Sichuan 611130 (CN)**
- **YU, Yan**
  **Chengdu, Sichuan 611130 (CN)**
- **ZHANG, Chen**
  **Chengdu, Sichuan 611130 (CN)**
- **YAN, Pangke**
  **Chengdu, Sichuan 611130 (CN)**

(74) Representative: **Biggi, Cristina**
**Bugnion S.p.A.**
**Viale Lancetti 17**
**20158 Milano (IT)**

# (54) TRICYCLIC FUSED HETEROCYCLIC PDE3/4 DUAL INHIBITOR AND USE THEREOF

(57) Disclosed in the present invention are a tricyclic fused heterocyclic compound having PDE3/4 dual inhibitory effect as represented by formula (I), and a stereoisomer, solvate, or pharmaceutically acceptable salt thereof, and the use thereof in the preparation of a drug for treating/preventing PDE3/4-mediated diseases, wherein each group in formula (I) is as defined in the description.

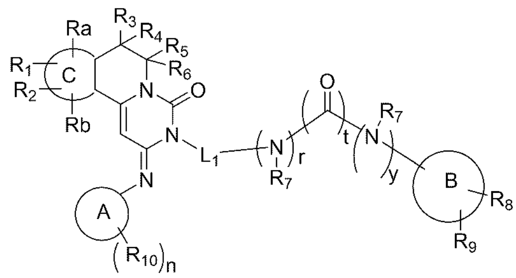

I

EP 4 467 543 A1

## Description

### Technical Field

The present invention relates to a PDE3/4 dual inhibitor and the use thereof in the preparation of a drug for the treatment of COPD and asthma.

### Background Art

COPD (chronic obstructive pulmonary disease) is a group of lung diseases characterized by airflow limitation that is not fully reversible and is progressive. It mainly affects the lungs and is the most common chronic killer of lung health. The incidence and mortality rates of COPD remain high. On the one hand, COPD is difficult to diagnose in its early stages and its onset is often hidden. Once the clinical manifestations of COPD appear, they often indicate a gradual decline in the patient's overall health and a gradual increase in respiratory symptoms. On the other hand, COPD is currently incurable, and drugs for treating COPD mainly rely on bronchodilators that can regulate airway smooth muscles. Such drugs can only relieve the symptoms and delay the worsening of the disease, but they only treat the symptoms and not the underlying cause. COPD has a long disease course, and patients often require frequent medical visits, hospitalization for acute exacerbations and long-term care, consuming large amounts of healthcare resources and becoming a challenging global disease burden. Therefore, new drugs for treating COPD need to be developed against different targets to meet various clinical needs.

Phosphodiesterase (PDE) is a member of a superfamily of enzyme systems that includes 11 families, each of which is involved in different signaling pathways and regulates different physiological processes. Studies have shown that PDE3 is associated with the contraction of smooth muscles in the respiratory system, while PDE4 plays a key role in the inflammatory response caused by immune cells. Given the clinical limitations of selective inhibitors of either PDE3 or PDE4, a PDE3/4 dual inhibitor appears to be a more attractive approach to target key pathological features of COPD and asthma. Currently, there is evidence that a PDE3/4 dual inhibitor has synergistic inhibitory effects, including synergistic anti-inflammatory and bronchodilatory effects. WO 2000058308 A1 reported that the compound RPL554 has long-acting bronchodilatory and anti-inflammatory effects, but the drug has poor solubility and high plasma clearance and is suitable for inhalation administration; in addition, biological activity data show that its PDE4 inhibitory activity is unsatisfactory, which may lead to suboptimal anti-inflammatory effects. Therefore, a PDE3/4 dual inhibitor is still worthy of further study.

### Summary of the Invention

The present invention provides a compound of formula (I), (II), (III) or (IV), or a stereoisomer, solvate, or pharmaceutically acceptable salt thereof, wherein the compound has the advantages of good activity, excellent physicochemical properties for preparation, high bioavailability, and low toxic and side effects.

The compound of formula (I), (II), (III) or (IV), or the stereoisomer, solvate, or pharmaceutically acceptable salt thereof,

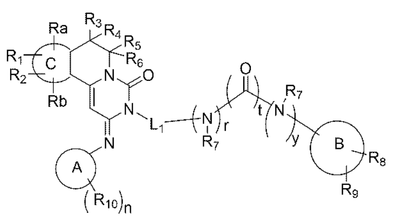

I ,

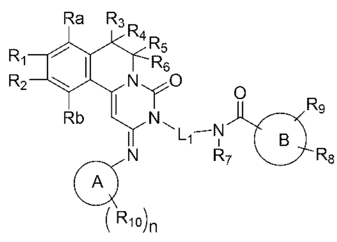

II ,

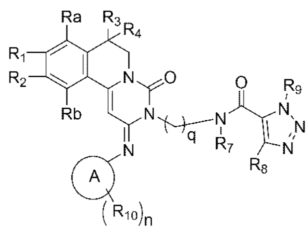

III

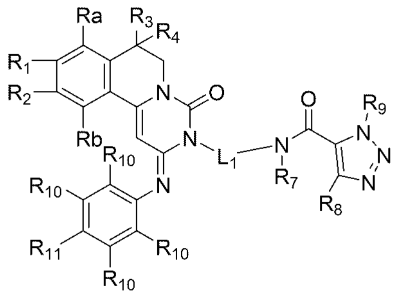

IV

wherein ring A is phenyl, a $C_{9-10}$ fused carbocycle or 5- to 6-membered heteroaryl containing 1-3 heteroatoms selected from N, S and O; in some embodiments, ring A is phenyl,

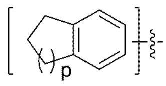

,

pyridyl, pyrazolyl, imidazolyl, pyrimidinyl, thienyl, thiazolyl, isothiazolyl, oxazolyl or isoxazolyl; in some embodiments, ring A is phenyl,

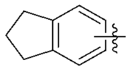

,

thienyl, imidazolyl, pyrazolyl, thiazolyl, isothiazolyl, oxazolyl or isoxazolyl; and in some embodiments, ring A is phenyl,

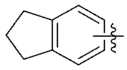

,

thienyl, or thiazolyl; and p is 1 or 2;

ring B is a 4- to 10-membered heterocycle containing 1-3 heteroatoms selected from N, S and O or a $C_{3-10}$ carbocycle; in some embodiments, ring B is phenyl, $C_{3-6}$cycloalkyl, 6-membered heteroaryl containing 1-3 heteroatoms selected from N, S and O, 4- to 7-membered heterocycloalkyl containing 1-3 heteroatoms selected from N, S and O, or 8- to 10-membered fused heterocycle or spirocyclic heterocycle containing 1-3 heteroatoms selected from N, S and O; in some embodiments, ring B is

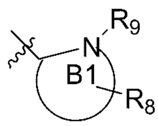

,

$B_1$ is a 5-membered heteroaryl containing 1-3 heteroatoms selected from N, S and O; in some embodiments, ring B is phenyl, $C_{3-6}$cycloalkyl, pyridyl, pyridazinyl, pyrazinyl, a 4- to 6-membered heterocycloalkyl containing 1-3 heteroatoms selected from N, S and O, or 8- to 10-membered fused heterocycle or spirocyclic heterocycle containing 1-3 heteroatoms selected from N, S and O; in some embodiments, ring B is

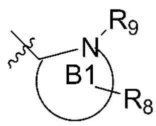

,

$B_1$ is triazazolyl, oxadiazolyl, imidazolyl or pyrrolyl; in some embodiments, ring B is

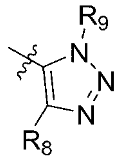 ;

and in some embodiments, ring B is phenyl, cyclopropyl, cyclobutyl, cyclopentyl, cyclohexyl, pyridazinyl, pyridyl, pyrimidinyl, pyranyl, piperidinyl, piperazinyl, azetidinyl, azolanyl, oxetanyl, oxolanyl, morpholinyl,

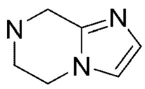 ,

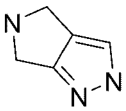 , 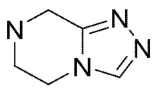 , 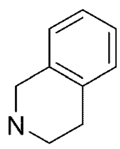 , 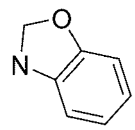 , 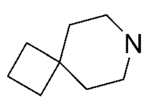 , 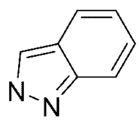 , 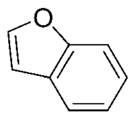 ,

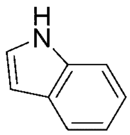 , 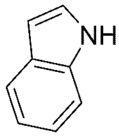 , 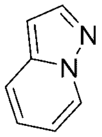 , 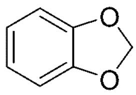 , 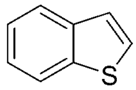 , 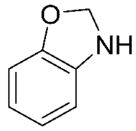 or 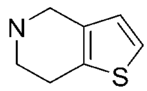 ;

ring C is phenyl or a 5- to 6-membered heteroaryl containing 1-3 heteroatoms selected from N, S and O; in some embodiments, ring C is phenyl, thienyl, imidazolyl, pyrrolyl, pyrazolyl, thiazolyl, pyridyl, pyrimidinyl or pyridazinyl; and in some embodiments, ring C is phenyl or thienyl;

$R_1$, $R_2$, $R_a$ and $R_b$ are independently H, deuterium, halogen, CN, OH, $NH_2$, - $NHC_{1-4}$alkyl, -N($C_{1-4}$alkyl)$_2$, -$OC_{1-4}$alkyl, -$O(CH_2)_mC_{3-6}$cycloalkyl, - $O(CH_2)_m$phenyl, -$O(CH_2)_m$-4- to 7-membered heterocycloalkyl containing 1-3 heteroatoms selected from N, S and O, -$O(CH_2)_m$-5- to 6-membered heteroaryl containing 1-3 heteroatoms selected from N, S and O, $C_{1-4}$alkyl, $C_{2-4}$alkenyl, $C_{2-4}$alkynyl, -$(CH_2)_mC_{3-6}$cycloalkyl, -$(CH_2)_m$phenyl, -$(CH_2)_m$-4- to 7-membered heterocycloalkyl containing 1-3 heteroatoms selected from N, S and O, and - $(CH_2)_m$-5- to 6-membered heteroaryl containing 1-3 heteroatoms selected from N, S and O or are not present, wherein the alkyl, phenyl, cycloalkyl, heterocycloalkyl and heteroaryl are optionally further substituted with 1-3 groups selected from deuterium, halogen, $C_{1-4}$alkyl, $C_{2-4}$alkenyl, $C_{2-4}$alkynyl, $C_{1-4}$alkoxy, deuterated $C_{1-4}$alkyl, deuterated $C_{1-4}$alkoxy, halogenated $C_{1-4}$alkoxy, CN, $NH_2$, -$NHC_{1-4}$alkyl, - N($C_{1-4}$alkyl)$_2$ and OH; in some embodiments, $R_1$, $R_2$, $R_a$ and $R_b$ are independently H, deuterium, halogen, CN, OH, $NH_2$, -$NHC_{1-4}$alkyl, -N($C_{1-4}$alkyl)$_2$, -$OC_{1-4}$alkyl, - $O(CH_2)_mC_{3-6}$cycloalkyl, -$O(CH_2)_m$phenyl, -$O(CH_2)_m$-4- to 7-membered heterocycloalkyl containing 1-3 heteroatoms selected from N, S and O, $C_{1-4}$alkyl, - $(CH_2)_mC_{3-6}$cycloalkyl, or -$(CH_2)_m$-4- to 7-membered heterocycloalkyl containing 1-3 heteroatoms selected from N, S and O, wherein the alkyl, cycloalkyl and heterocycloalkyl are optionally further substituted with 1-3 groups selected from deuterium, halogen, $C_{1-4}$alkyl, $C_{2-4}$alkenyl, $C_{2-4}$alkynyl, $C_{1-4}$alkoxy, deuterated $C_{1-4}$alkyl, deuterated $C_{1-4}$alkoxy and halogenated $C_{1-4}$alkoxy; in some embodiments, $R_1$, $R_2$, $R_a$ and $R_b$ are independently H, deuterium, halogen, CN, OH, -$OC_{1-4}$alkyl, - $O(CH_2)_mC_{3-6}$cycloalkyl, -$O(CH_2)_m$phenyl , $C_{1-4}$alkyl or -$(CH_2)_mC_{3-6}$cycloalkyl, wherein the alkyl and cycloalkyl are optionally further substituted with 1-3 groups selected from deuterium, halogen, $C_{1-4}$alkyl, $C_{1-4}$alkoxy, deuterated $C_{1-4}$alkyl, deuterated $C_{1-4}$alkoxy and halogenated $C_{1-4}$alkoxy; and in some embodiments, $R_1$, $R_2$, $R_a$ and $R_b$ are independently H, methoxy, ethoxy, propoxy, isopropoxy, - $O(CH_2)_m$cyclopropyl, benzyloxy, methyl, ethyl, propyl, isopropyl or - $(CH_2)_m$cyclopropyl, wherein the methoxy, ethoxy, propoxy, isopropoxy, benzyloxy, methyl, ethyl, propyl, isopropyl and cyclopropyl are optionally further substituted with 1-3 groups selected from deuterium, F, Cl, Br, I, methyl, ethyl, propyl, isopropyl, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy; and each m is independently 0, 1, 2, 3 or 4;

optionally, $R_1$ and $R_2$ together with the atom to which they are attached form a 5- to 7-membered heterocycle containing 1-3 heteroatoms selected from N, S and O, or a $C_{4-7}$ carbocycle, wherein the heterocycle and carbocycle are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy; in some embodiments, $R_1$ and $R_2$ together with the atom to which they are attached form a 5- to 6-membered heterocycle containing 1-3

heteroatoms selected from N, S and O, or a $C_{5-6}$ carbocycle, wherein the heterocycle and carbocycle are optionally substituted with 1-3 groups selected from deuterium, halogen, $C_{1-4}$alkyl, halogenated $C_{1-4}$alkyl, $C_{1-4}$alkoxy, deuterated $C_{1-4}$alkoxy and halogenated $C_{1-4}$alkoxy; in some embodiments, $R_1$ and $R_2$ together with the atom to which they are attached form a 5- to 6-membered heterocycle containing 1-3 heteroatoms selected from N, S and O, or a $C_{5-6}$ carbocycle, wherein the heterocycle and carbocycle are optionally substituted with 1-3 groups selected from deuterium, halogen, $C_{1-4}$alkyl, halogenated $C_{1-4}$alkyl and $C_{1-4}$alkoxy; and in some embodiments, R, and $R_2$ together with the atom to which they are attached form

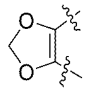,

and are optionally substituted with 1-2 groups selected from deuterium, F, Cl, Br, I, methyl, ethyl, propyl, methoxy, ethoxy, propoxy, halogenated methyl, halogenated ethyl and halogenated propyl;

$R_3$, $R_4$, $R_5$ and $R_6$ are independently H, deuterium, halogen, $C_{1-4}$alkyl, - $(CH_2)_mC_{3-6}$cycloalkyl, wherein the alkyl and cycloalkyl are optionally substituted with 1-3 groups selected from deuterium, halogen, CN and $NH_2$; in some embodiments, $R_3$, $R_4$, Rs and $R_6$ are independently H, deuterium, halogen, $C_{1-4}$alkyl, $-(CH_2)_mC_{3-6}$cycloalkyl, wherein the alkyl and cycloalkyl are optionally substituted with 1-3 groups selected from deuterium, halogen, CN and $NH_2$; in some embodiments, $R_3$, $R_4$, $R_5$ and $R_6$ are independently H, deuterium, halogen and $C_{1-4}$alkyl, wherein the alkyl is optionally substituted with 1-3 groups selected from deuterium, halogen, CN and $NH_2$; in some embodiments, $R_3$ and $R_4$ are independently H, deuterium, halogen and $C_{1-4}$alkyl, wherein the alkyl is optionally substituted with 1-3 groups selected from deuterium, F, Cl, Br and I; and $R_5$ and $R_6$ are H; and in some embodiments, $R_3$ and $R_4$ are independently H, deuterium, F, Cl, Br, I, methyl, ethyl or propyl, wherein the methyl, ethyl or propyl is optionally substituted with 1-3 groups selected from deuterium, F, Cl, Br and I; $R_5$ and $R_6$ are H; and each m is independently 0, 1, 2, 3 or 4;

optionally, $R_3$ and $R_4$, or $R_5$ and $R_6$ together with the carbon atom to which they are attached form $C_{3-6}$cycloalkyl;

$L_1$ is $C_{1-6}$alkylene, wherein the alkylene is optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy; in some embodiments, $L_1$ is $C_{2-4}$alkylene, wherein the alkylene is optionally substituted with 1-3 groups selected from deuterium, halogen, $C_{1-4}$alkoxy, deuterated $C_{1-4}$alkoxy and halogenated $C_{1-4}$alkoxy; and in some embodiments, $L_1$ is $-(CH_2)q$;

each $R_7$ is independently H, $C_{1-4}$alkyl or $-(CH_2)_mC_{3-6}$cycloalkyl, wherein the alkyl and cycloalkyl are optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy; in some embodiments, $R_7$ is H or $C_{1-4}$alkyl, wherein the alkyl is optionally substituted with 1-3 groups selected from deuterium, halogen, $C_{1-4}$alkoxy, deuterated $C_{1-4}$alkoxy and halogenated $C_{1-4}$alkoxy; in some embodiments, $R_7$ is H or $C_{1-3}$alkyl, wherein the alkyl is optionally substituted with 1-3 groups selected from deuterium, halogen, $C_{1-4}$alkoxy, deuterated $C_{1-4}$alkoxy and halogenated $C_{1-4}$alkoxy; and in some embodiments, $R_7$ is H, methyl, ethyl, propyl or isopropyl, wherein the methyl, ethyl, propyl or isopropyl is optionally substituted with 1-3 groups selected from deuterium, F, Cl, Br, I, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy; and each m is independently 0, 1, 2, 3 or 4;

$R_8$ and $R_9$ are independently H, =O, $C_{1-4}$alkyl, OH, $-OC_{1-4}$alkyl, $-NHC_{1-4}$alkyl, $-N(C_{1-4}alkyl)_2$, $-C(O)C_{1-4}$alkyl, $-C(O)NH_2$, $-C(O)NHC_{1-4}$alkyl, $-C(O)N(C_{1-4}alkyl)_2$, CN, halogen and $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy;

in some embodiments, ring B is phenyl, $C_{3-6}$cycloalkyl, 6-membered heteroaryl containing 1-3 heteroatoms selected from N, S and O, 4- to 7-membered heterocycloalkyl containing 1-3 heteroatoms selected from N, S and O, or 8- to 10-membered fused heterocycle or spirocyclic heterocycle containing 1-3 heteroatoms selected from N, S and O, and $R_8$ and $R_9$ are independently H, =O, $C_{1-4}$alkyl, OH, - $NHC_{1-4}$alkyl, $-N(C_{1-4}alkyl)_2$, $-C(O)C_{1-4}$alkyl, $-C(O)NH_2$, $-C(O)NHC_{1-4}$alkyl, - $C(O)N(C_{1-4}alkyl)_2$, CN, halogen and $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy;

in some embodiments, ring B is phenyl, $C_{3-6}$cycloalkyl, pyridyl, pyridazinyl, pyrazinyl, 4- to 6-membered heterocycloalkyl containing 1-3 heteroatoms selected from N, S and O, or 8- to 10-membered fused heterocycle or spirocyclic heterocycle containing 1-3 heteroatoms selected from N, S and O, and $R_8$ and $R_9$ are independently H, =O, $C_{1-4}$alkyl, OH, $-NHC_{1-4}$alkyl, $-N(C_{1-4}alkyl)_2$, $-C(O)C_{1-4}$alkyl, $-C(O)NH_2$, $-C(O)NHC_{1-4}$alkyl, $-C(O)N(C_{1-4}alkyl)_2$, CN, halogen and $C_{3-6}$cycloalkyl, wherein the alkyl and cycloalkyl are optionally substituted with 1-3 groups selected from deuterium, halogen, $C_{1-4}$alkyl, and halogenated $C_{1-4}$alkyl;

in some embodiments, ring B is

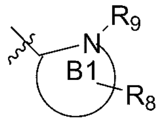

,

and $B_1$ is a 5-membered heteroaryl containing 1-3 heteroatoms selected from N, S and O; $R_8$ is H, $C_{1-4}$alkyl, OH, CN, halogen or $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy; and $R_9$ is $C_{1-4}$alkyl or $C_{3-6}$cycloalkyl, wherein the alkyl and cycloalkyl are optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy;

in some embodiments, ring B is

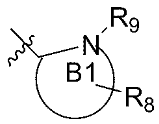

,

and $B_1$ is triazazolyl, oxadiazolyl, imidazolyl or pyrrolyl; $R_8$ is H, OH, $C_{1-4}$alkyl and $C_{3-6}$cycloalkyl, wherein the alkyl and cycloalkyl are optionally substituted with 1-3 groups selected from deuterium, halogen, $C_{1-4}$alkoxy, deuterated $C_{1-4}$alkoxy and halogenated $C_{1-4}$alkoxy; and $R_9$ is $C_{1-4}$alkyl or $C_{3-6}$cycloalkyl, wherein the alkyl and cycloalkyl are optionally substituted with 1-3 groups selected from deuterium, halogen, $C_{1-4}$alkoxy, deuterated $C_{1-4}$alkoxy and halogenated $C_{1-4}$alkoxy;

in some embodiments, ring B is

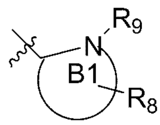

,

and $R_8$ is H, OH or $C_{1-4}$alkyl, wherein the alkyl is optionally substituted with 1-3 groups selected from deuterium, halogen, $C_{1-4}$alkoxy and halogenated $C_{1-4}$alkoxy; and $R_9$ is $C_{1-4}$alkyl or $C_{3-6}$cycloalkyl, wherein the alkyl and cycloalkyl are optionally substituted with 1-3 groups selected from deuterium, halogen, $C_{1-4}$alkoxy and halogenated $C_{1-4}$alkoxy;

each $R_{10}$ is independently H, halogen, CN, $NH_2$, $-NHC_{1-4}$alkyl, $-N(C_{1-4}alkyl)_2$, OH, $C_{1-4}$alkyl, $C_{2-4}$alkenyl, $C_{2-4}$alkynyl, $-(CH_2)_m-C_{3-6}$cycloalkyl, $-O-C_{3-6}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl, $C_{2-4}$alkenyl, $C_{2-4}$alkynyl, CN, $NH_2$, $-NHC_{1-4}$alkyl, $-N(C_{1-4}alkyl)_2$ and OH; in some embodiments, each $R_{10}$ is independently H, halogen, CN, $C_{1-4}$alkyl, $C_{2-4}$alkenyl, $C_{2-4}$alkynyl, $C_{3-6}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl and CN; in some embodiments, each $R_{10}$ is independently H, halogen, CN, $C_{1-4}$alkyl, $C_{2-4}$alkenyl, $C_{2-4}$alkynyl, $C_{3-4}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl and CN; in some embodiments, each $R_{10}$ is independently H, F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy, wherein the methyl, ethyl, propyl, isopropyl, n-butyl, isobutyl, ethenyl, propenyl, allyl, ethynyl, propynyl, cyclopropyl, cyclobutyl, methoxy, ethoxy and propoxy are optionally substituted with 1-3 groups selected from deuterium, F, Cl, Br, I, methyl, ethyl, propyl and CN; and each m is independently 0, 1, 2, 3 or 4;

$R_{11}$ is H, halogen, CN, $NH_2$, $-NHC_{1-4}$alkyl, $-N(C_{1-4}alkyl)_2$, OH, $C_{1-4}$alkyl, $C_{2-4}$alkenyl, $C_{2-4}$alkynyl, $-(CH_2)_m-C_{3-6}$cycloalkyl, $-O-C_{3-6}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl, $C_{2-4}$alkenyl, $C_{2-4}$alkynyl, CN, $NH_2$, $-NHC_{1-4}$alkyl, $-N(C_{1-4}alkyl)_2$ and OH; in some embodiments, $R_{11}$ is H, halogen, CN, $C_{1-4}$alkyl, $C_{2-4}$alkenyl, $C_{2-4}$alkynyl, $C_{3-6}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl and CN; in some embodiments, $R_{11}$ is H, halogen, CN, $C_{1-4}$alkyl, $C_{2-4}$alkenyl, $C_{2-4}$alkynyl, $C_{3-4}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl and CN; and in some embodiments, $R_{11}$ is H, F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy, wherein the methyl, ethyl, propyl, isopropyl, n-butyl,

isobutyl, ethenyl, propenyl, allyl, ethynyl, propynyl, cyclopropyl, cyclobutyl, methoxy, ethoxy and propoxy are optionally substituted with 1-3 groups selected from deuterium, F, Cl, Br, I, methyl, ethyl, propyl and CN;

when $R_1$ and $R_2$ are both methoxy, $R_{11}$ is F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy; in some embodiments, when $R_1$ and $R_2$ are both methoxy, $R_{11}$ is F, Cl, ethenyl, propenyl, allyl, ethynyl, propynyl, cyclopropyl, cyclobutyl, methoxy, ethoxy or propoxy; and in some embodiments, when $R_1$ and $R_2$ are both methoxy, $R_{11}$ is cyclopropyl, cyclobutyl, methoxy, ethoxy or propoxy;

n is 0, 1, 2, 3 or 4; in some embodiments, n is 1, 2 or 3; and in some embodiments, n is 2 or 3;

r, t and y are independently 0 or 1;

q is 1, 2 or 3; and in some embodiments, q is 2 or 3;

each m is independently 0, 1, 2, or 3; and in some embodiments, each m is independently 0, 1 or 2;

p is 1 or 2; and

q is 1, 2 or 3; and in some embodiments, q is 2 or 3.

The compound of the present invention is not

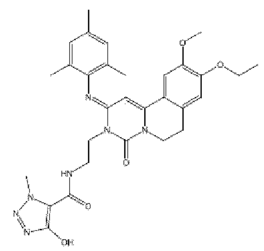

More specifically, the first technical solution of the present invention provides a compound of formula (I), or a stereoisomer, solvate, or pharmaceutically acceptable salt thereof,

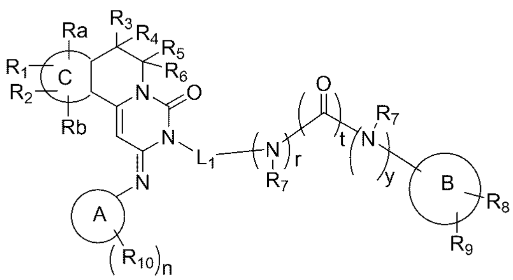

I

wherein ring A is phenyl, a $C_{9-10}$ fused carbocycle or 5- to 6-membered heteroaryl containing 1-3 heteroatoms selected from N, S and O;

ring B is a 4- to 10-membered heterocycle containing 1-3 heteroatoms selected from N, S and O or a $C_{3-10}$ carbocycle;

ring C is phenyl or a 5- to 6-membered heteroaryl containing 1-3 heteroatoms selected from N, S and O;

$R_1$, $R_2$, $R_a$ and $R_b$ are independently H, deuterium, halogen, CN, OH, $NH_2$, $-NHC_{1-4}$alkyl, $-N(C_{1-4}alkyl)_2$, $-OC_{1-4}$alkyl, $-O(CH_2)_mC_{3-6}$cycloalkyl, $-O(CH_2)_m$phenyl, $-O(CH_2)_m$-4- to 7-membered heterocycloalkyl containing 1-3 heteroatoms selected from N, S and O, $-O(CH_2)_m$-5- to 6-membered heteroaryl containing 1-3 heteroatoms selected from N, S and O, $C_{1-4}$alkyl, $C_{2-4}$alkenyl, $C_{2-4}$alkynyl, $-(CH_2)_mC_{3-6}$cycloalkyl, $-(CH_2)_m$phenyl, $-(CH_2)_m$-4- to 7-membered heterocycloalkyl containing 1-3 heteroatoms selected from N, S and O, and $-(CH_2)_m$-5- to 6-membered heteroaryl containing 1-3 heteroatoms selected from N, S and O or are not present, wherein the alkyl, phenyl, cycloalkyl, heterocycloalkyl and heteroaryl are optionally further substituted with 1-3 groups selected from deuterium, halogen, $C_{1-4}$alkyl, $C_{2-4}$alkenyl, $C_{2-4}$alkynyl, $C_{1-4}$alkoxy, deuterated $C_{1-4}$alkyl, deuterated $C_{1-4}$alkoxy, halogenated $C_{1-4}$alkoxy, CN, $NH_2$, $-NHC_{1-4}$alkyl, $-N(C_{1-4}alkyl)_2$ and OH;

optionally, $R_1$ and $R_2$ together with the atom to which they are attached form a 5- to 7-membered heterocycle containing 1-3 heteroatoms selected from N, S and O, or a $C_{4-7}$ carbocycle, wherein the heterocycle and carbocycle are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy;

$R_3$, $R_4$, $R_5$ and $R_6$ are independently H, deuterium, halogen, $C_{1-4}$alkyl, $-(CH_2)_mC_{3-6}$cycloalkyl, wherein the alkyl and cycloalkyl are optionally substituted with 1-3 groups selected from deuterium, halogen, CN and $NH_2$;

optionally, $R_3$ and $R_4$, or $R_5$ and $R_6$ together with the carbon atom to which they are attached form $C_{3-6}$cycloalkyl;
$L_1$ is $C_{1-6}$alkylene, wherein the alkylene is optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy;
each $R_7$ is independently H, $C_{1-4}$alkyl or $-(CH_2)_mC_{3-6}$cycloalkyl, wherein the alkyl and cycloalkyl are optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy;
$R_8$ and $R_9$ are independently H, =O, $C_{1-4}$alkyl, OH, $-OC_{1-4}$alkyl, $-NHC_{1-4}$alkyl, $-N(C_{1-4}alkyl)_2$, $-C(O)C_{1-4}$alkyl, $-C(O)NH_2$, $-C(O)NHC_{1-4}$alkyl, $-C(O)N(C_{1-4}alkyl)_2$, CN, halogen and $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy;
each $R_{10}$ is independently H, halogen, CN, $NH_2$, $-NHC_{1-4}$alkyl, $-N(C_{1-4}alkyl)_2$, OH, $C_{1-4}$alkyl, $C_{2-4}$alkenyl, $C_{2-4}$alkynyl, $-(CH_2)_m-C_{3-6}$cycloalkyl, $-O-C_{3-6}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl, $C_{2-4}$alkenyl, $C_{2-4}$alkynyl, CN, $NH_2$, $-NHC_{1-4}$alkyl, $-N(C_{1-4}alkyl)_2$ and OH;
n is 0, 1, 2, 3 or 4;
each m is independently 0, 1, 2, 3 or 4; and
r, t and y are independently 0 or 1.

More specifically, the second technical solution of the present invention provides a compound of formula I, or a stereoisomer, solvate, or pharmaceutically acceptable salt thereof, wherein the compound has a structure of formula II:

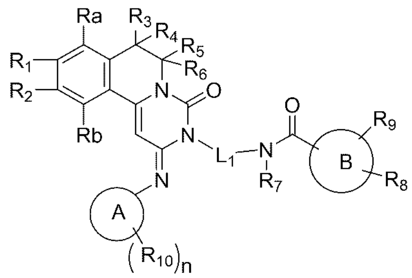

II

wherein, (1) ring B is: phenyl, $C_{3-6}$cycloalkyl, 6-membered heteroaryl containing 1-3 heteroatoms selected from N, S and O, 4- to 7-membered heterocycloalkyl containing 1-3 heteroatoms selected from N, S and O, or 8- to 10-membered fused heterocycle or spirocyclic heterocycle containing 1-3 heteroatoms selected from N, S and O; and
Rs and $R_9$ are independently H, =O, $C_{1-4}$alkyl, OH, $-NHC_{1-4}$alkyl, $-N(C_{1-4}alkyl)_2$, $-C(O)C_{1-4}$alkyl, $-C(O)NH_2$, $-C(O)NHC_{1-4}$alkyl, $-C(O)N(C_{1-4}alkyl)_2$, CN, halogen and $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy; or
(2) ring B is:

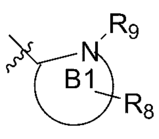
,

and $B_1$ is a 5-membered heteroaryl containing 1-3 heteroatoms selected from N, S and O;
$R_8$ is H, $C_{1-4}$alkyl, OH, CN, halogen or $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy; and
$R_9$ is $C_{1-4}$alkyl or $C_{3-6}$cycloalkyl, wherein the alkyl and cycloalkyl are optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy;
provided that the compound is not

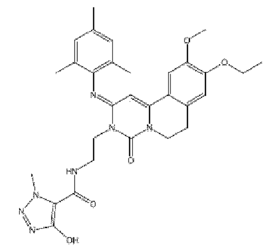
;

and
the remaining groups are as described in the first technical solution.

More specifically, the third technical solution of the present invention provides a compound of formula II, or a stereoisomer, solvate, or pharmaceutically acceptable salt thereof,

wherein ring A is phenyl,

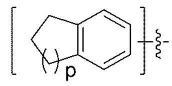
,

pyridyl, pyrazolyl, pyrimidinyl, thienyl, thiazolyl, isothiazolyl, oxazolyl or isoxazolyl;
$R_1$, $R_2$, $R_a$ and $R_b$ are independently H, deuterium, halogen, CN, OH, $NH_2$, - $NHC_{1-4}$alkyl, -$N(C_{1-4}$alkyl$)_2$, -$OC_{1-4}$alkyl, -$O(CH_2)_mC_{3-6}$cycloalkyl, - $O(CH_2)_m$phenyl, -$O(CH_2)_m$-4- to 7-membered heterocycloalkyl containing 1-3 heteroatoms selected from N, S and O, $C_{1-4}$alkyl, -$(CH_2)_mC_{3-6}$cycloalkyl, or - $(CH_2)_m$-4- to 7-membered heterocycloalkyl containing 1-3 heteroatoms selected from N, S and O, wherein the alkyl, cycloalkyl and heterocycloalkyl are optionally further substituted with 1-3 groups selected from deuterium, halogen, $C_{1-4}$alkyl, $C_{2-4}$alkenyl, $C_{2-4}$alkynyl, $C_{1-4}$alkoxy, deuterated $C_{1-4}$alkyl, deuterated $C_{1-4}$alkoxy and halogenated $C_{1-4}$alkoxy;
optionally, $R_1$ and $R_2$ together with the atom to which they are attached form a 5- to 6-membered heterocycle containing 1-3 heteroatoms selected from N, S and O, or a $C_{5-6}$ carbocycle, wherein the heterocycle and carbocycle are optionally substituted with 1-3 groups selected from deuterium, halogen, $C_{1-4}$alkyl, halogenated $C_{1-4}$alkyl, $C_{1-4}$alkoxy, deuterated $C_{1-4}$alkoxy and halogenated $C_{1-4}$alkoxy;
$R_3$, $R_4$, $R_5$ and $R_6$ are independently H, deuterium, halogen and $C_{1-4}$alkyl, wherein the alkyl is optionally substituted with 1-3 groups selected from deuterium, halogen, CN and $NH_2$;
$L_1$ is $C_{2-4}$alkylene, wherein the alkylene is optionally substituted with 1-3 groups selected from deuterium, halogen, $C_{1-4}$alkoxy, deuterated $C_{1-4}$alkoxy and halogenated $C_{1-4}$alkoxy;
$R_7$ is H or $C_{1-4}$alkyl, wherein the alkyl is optionally substituted with 1-3 groups selected from deuterium, halogen, $C_{1-4}$alkoxy, deuterated $C_{1-4}$ alkoxy and halogenated $C_{1-4}$alkoxy;
each $R_{10}$ is independently H, halogen, CN, $C_{1-4}$alkyl, $C_{2-4}$alkenyl, $C_{2-4}$alkynyl, $C_{3-6}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl and CN;
n is 1, 2 or 3;
each m is independently 0, 1, 2, or 3;
p is 1 or 2;
and

(1) ring B is phenyl, $C_{3-6}$cycloalkyl, pyridyl, pyridazinyl, pyrazinyl, a 4- to 6-membered heterocycloalkyl containing 1-3 heteroatoms selected from N, S and O, or 8- to 10-membered fused heterocycle or spirocyclic heterocycle containing 1-3 heteroatoms selected from N, S and O; and
Rs and $R_9$ are independently H, =O, $C_{1-4}$alkyl, OH, -$NHC_{1-4}$alkyl, -$N(C_{1-4}$alkyl$)_2$, -$C(O)C_{1-4}$alkyl, -$C(O)NH_2$, -$C(O)NHC_{1-4}$alkyl, -$C(O)N(C_{1-4}$alkyl$)_2$, CN, halogen and $C_{3-6}$cycloalkyl, wherein the alkyl and cycloalkyl are optionally substituted with 1-3 groups selected from deuterium, halogen, $C_{1-4}$alkyl and halogenated $C_{1-4}$alkyl; or

(2) ring B is

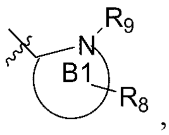

and $B_1$ is triazazolyl, oxadiazolyl, imidazolyl or pyrrolyl;

$R_8$ is H, OH, $C_{1-4}$alkyl and $C_{3-6}$cycloalkyl, wherein the alkyl and cycloalkyl are optionally substituted with 1-3 groups selected from deuterium, halogen, $C_{1-4}$alkoxy, deuterated $C_{1-4}$alkoxy and halogenated $C_{1-4}$alkoxy; and

$R_9$ is $C_{1-4}$alkyl or $C_{3-6}$cycloalkyl, wherein the alkyl and cycloalkyl are optionally substituted with 1-3 groups selected from deuterium, halogen, $C_{1-4}$alkoxy, deuterated $C_{1-4}$alkoxy and halogenated $C_{1-4}$alkoxy; and

the remaining groups are as described in the second technical solution.

More specifically, the fourth technical solution of the present invention provides a compound of formula II, or a stereoisomer, solvate, or pharmaceutically acceptable salt thereof, wherein the compound has a structure of formula III:

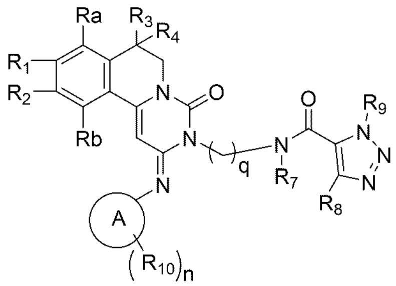

III

wherein $R_1$, $R_2$, $R_a$ and $R_b$ are independently H, deuterium, halogen, CN, OH, -O$C_{1-4}$alkyl, -O$(CH_2)_m$$C_{3-6}$cycloalkyl, -O$(CH_2)_m$phenyl , $C_{1-4}$alkyl or -$(CH_2)_m$$C_{3-6}$cycloalkyl, wherein the alkyl and cycloalkyl are optionally further substituted with 1-3 groups selected from deuterium, halogen, $C_{1-4}$alkyl, $C_{1-4}$alkoxy, deuterated $C_{1-4}$alkyl, deuterated $C_{1-4}$alkoxy and halogenated $C_{1-4}$alkoxy;

optionally, $R_1$ and $R_2$ together with the atom to which they are attached form a 5- to 6-membered heterocycle containing 1-3 heteroatoms selected from N, S and O, or a $C_{5-6}$ carbocycle, wherein the heterocycle and carbocycle are optionally substituted with 1-3 groups selected from deuterium, halogen, $C_{1-4}$alkyl, halogenated $C_{1-4}$alkyl and $C_{1-4}$alkoxy;

$R_3$ and $R_4$ are independently H, deuterium, halogen and $C_{1-4}$alkyl, wherein the alkyl is optionally substituted with 1-3 groups selected from deuterium, F, Cl, Br and I;

ring A is phenyl,

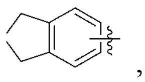

thienyl, pyrazolyl, thiazolyl, isothiazolyl, oxazolyl or isoxazolyl;

$R_7$ is H or $C_{1-3}$ alkyl, wherein the alkyl is optionally substituted with 1-3 groups selected from deuterium, halogen, $C_{1-4}$ alkoxy, deuterated $C_{1-4}$ alkoxy and halogenated $C_{1-4}$ alkoxy;

$R_8$ is H, OH or $C_{1-4}$alkyl, wherein the alkyl is optionally substituted with 1-3 groups selected from deuterium, halogen, $C_{1-4}$alkoxy and halogenated $C_{1-4}$alkoxy;

$R_9$ is $C_{1-4}$alkyl or $C_{3-6}$cycloalkyl, wherein the alkyl and cycloalkyl are optionally substituted with 1-3 groups selected from deuterium, halogen, $C_{1-4}$alkoxy and halogenated $C_{1-4}$alkoxy;

each $R_{10}$ is independently H, halogen, CN, $C_{1-4}$alkyl, $C_{2-4}$alkenyl, $C_{2-4}$alkynyl, $C_{3-4}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl and CN;

n is 2 or 3;

each m is independently 0, 1, or 2;

q is 1, 2 or 3; and

the remaining groups are as described in the third technical solution.

More specifically, the fifth technical solution of the present invention provides a compound of formula III, or a stereoisomer, solvate, or pharmaceutically acceptable salt thereof, wherein $R_1$, $R_2$, $R_a$ and $R_b$ are independently H, methoxy, ethoxy, propoxy, isopropoxy, $-O(CH_2)_m$cyclopropyl, benzyloxy, methyl, ethyl, propyl, isopropyl or $-(CH_2)_m$cyclopropyl, wherein the methoxy, ethoxy, propoxy, isopropoxy, benzyloxy, methyl, ethyl, propyl, isopropyl and cyclopropyl are optionally further substituted with 1-3 groups selected from deuterium, F, Cl, Br, I, methyl, ethyl, propyl, isopropyl, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy;

optionally, $R_1$ and $R_2$ together with the atom to which they are attached form

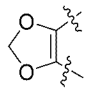,

and are optionally substituted with 1-2 groups selected from deuterium, F, Cl, Br, I, methyl, ethyl, propyl, methoxy, ethoxy, propoxy, halogenated methyl, halogenated ethyl and halogenated propyl;
$R_3$ and $R_4$ are independently H, deuterium, F, Cl, Br, I, methyl, ethyl or propyl, wherein the methyl, ethyl or propyl is optionally substituted with 1-3 groups selected from deuterium, F, Cl, Br and I;
ring A is phenyl,

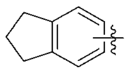,

thienyl or thiazolyl;
$R_7$ is H, methyl, ethyl, propyl or isopropyl, wherein the methyl, ethyl, propyl or isopropyl is optionally substituted with 1-3 groups selected from deuterium, F, Cl, Br, I, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy;
$R_8$ is H, OH, methyl, ethyl, propyl or isopropyl, wherein the methyl, ethyl, propyl or isopropyl is optionally substituted with 1-3 groups selected from deuterium, halogen, methoxy, ethoxy, propoxy, halogenated methoxy, halogenated ethoxy and halogenated propoxy;
$R_9$ is methyl, ethyl, propyl, isopropyl, cyclopropyl, cyclobutyl or cyclopentyl, wherein the methyl, ethyl, propyl, isopropyl, cyclopropyl, cyclobutyl or cyclopentyl is optionally substituted with 1-3 groups selected from deuterium, halogen, methoxy, ethoxy, propoxy, halogenated methoxy, halogenated ethoxy and halogenated propoxy;
each $R_{10}$ is independently H, F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy, wherein the methyl, ethyl, propyl, isopropyl, n-butyl, isobutyl, ethenyl, propenyl, allyl, ethynyl, propynyl, cyclopropyl, cyclobutyl, methoxy, ethoxy and propoxy are optionally substituted with 1-3 groups selected from deuterium, F, Cl, Br, I, methyl, ethyl, propyl and CN;
q is 2 or 3; and
the remaining groups are as described in the fourth technical solution.

More specifically, the sixth technical solution of the present invention provides a compound of formula II, or a stereoisomer, solvate, or pharmaceutically acceptable salt thereof, wherein the compound has a structure of formula IV:

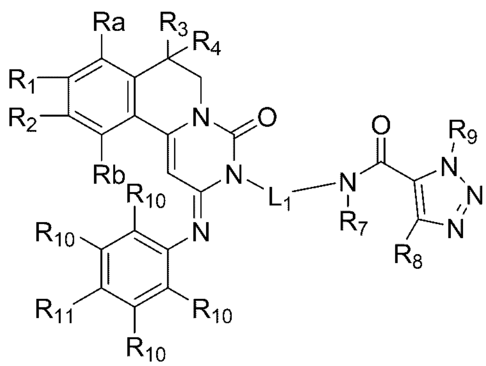

IV

wherein $R_1$, $R_2$, $R_a$ and $R_b$ are independently H, methoxy, ethoxy, propoxy, isopropoxy, $-O(CH_2)_m$cyclopropyl, benzyloxy, methyl, ethyl, propyl, isopropyl or - $(CH_2)_m$cyclopropyl, wherein the methoxy, ethoxy, propoxy, isopropoxy, benzyloxy, methyl, ethyl, propyl, isopropyl and cyclopropyl are optionally further substituted with 1-3 groups selected from deuterium, F, Cl, Br, I, methyl, ethyl, propyl, isopropyl, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy;

$R_3$ and $R_4$ are independently H, deuterium, F, Cl, Br, I, methyl, ethyl or propyl, wherein the methyl, ethyl or propyl is optionally substituted with 1-3 groups selected from deuterium, F, Cl, Br and I;

$L_1$ is $-CH_2CH_2-$ or $-CH_2CH_2CH_2-$;

$R_7$ is H, methyl, ethyl, propyl or isopropyl, wherein the methyl, ethyl, propyl or isopropyl is optionally substituted with 1-3 groups selected from deuterium, F, Cl, Br, I, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy;

$R_8$ is H, OH, methyl, ethyl, propyl or isopropyl, wherein the methyl, ethyl, propyl or isopropyl is optionally substituted with 1-3 groups selected from deuterium, halogen, methoxy, ethoxy, propoxy, halogenated methoxy, halogenated ethoxy and halogenated propoxy;

$R_9$ is methyl, ethyl, propyl, isopropyl, cyclopropyl, cyclobutyl or cyclopentyl, wherein the methyl, ethyl, propyl, isopropyl, cyclopropyl, cyclobutyl or cyclopentyl is optionally substituted with 1-3 groups selected from deuterium, halogen, methoxy, ethoxy, propoxy, halogenated methoxy, halogenated ethoxy and halogenated propoxy; and

each $R_{10}$ and $R_{11}$ is independently H, F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy, wherein the methyl, ethyl, propyl, isopropyl, n-butyl, isobutyl, ethenyl, propenyl, allyl, ethynyl, propynyl, cyclopropyl, cyclobutyl, methoxy, ethoxy and propoxy are optionally substituted with 1-3 groups selected from deuterium, F, Cl, Br, I, methyl, ethyl, propyl and CN;

provided that when $R_1$ and $R_2$ are both methoxy, $R_{11}$ is F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy.

The remaining groups are as described in the second technical solution.

More specifically, the seventh technical solution of the present invention provides a compound of formula I, or a stereoisomer, solvate, or pharmaceutically acceptable salt thereof, wherein the compound is selected from one of the following structures:

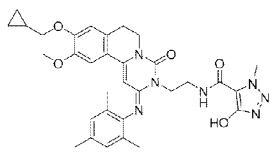 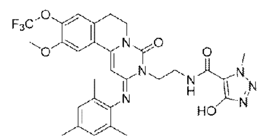 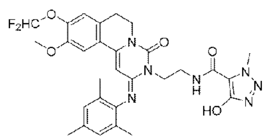 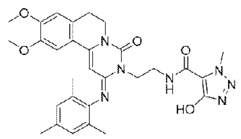

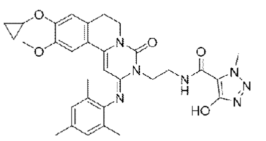 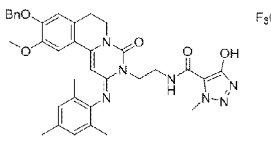 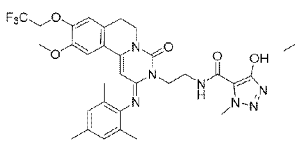 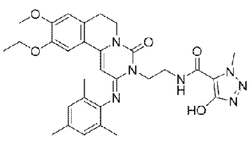

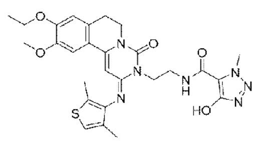 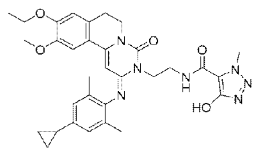 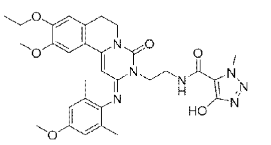 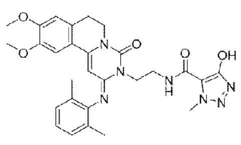

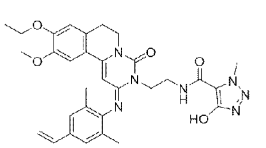 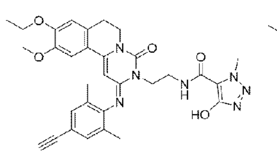 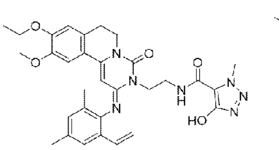 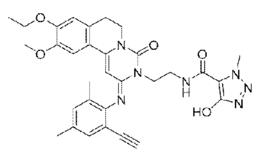

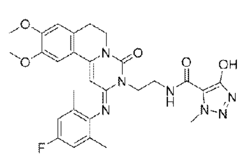 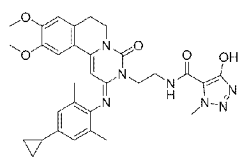 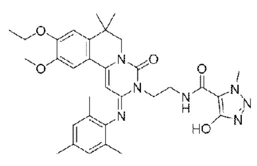 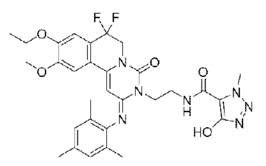

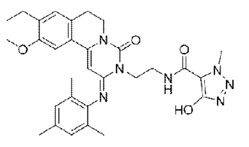 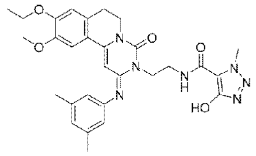 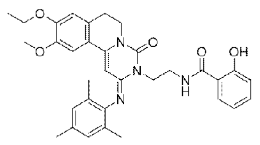 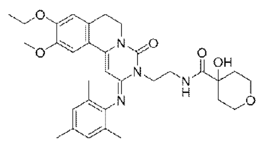

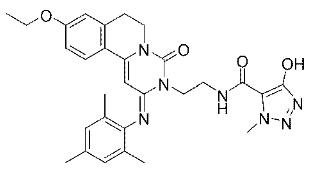

More specifically, the eighth technical solution of the present invention provides a pharmaceutical composition comprising the compound, or the stereoisomer, solvate, or pharmaceutically acceptable salt thereof according to any one of the first to the sixth technical solutions, and a pharmaceutically acceptable carrier and/or excipient.

More specifically, the ninth technical solution of the present invention provides the use of the compound, or the stereoisomer, solvate, or pharmaceutically acceptable salt thereof according to any one of the first to the seventh technical solutions, or the composition according to the eighth technical solution in the preparation of a drug for the treatment/-prevention of PDE3/4-mediated diseases.

Further, the PDE3/4-mediated diseases are selected from COPD and asthma.

The present invention further provides a composition or pharmaceutical preparation comprising the compound, or the stereoisomer or pharmaceutically acceptable salt thereof according to any one of the preceding solutions, and a pharmaceutically acceptable carrier and/or excipient. The pharmaceutical composition may be in a unit preparation form (the unit preparation is also referred to as the "preparation strength").

Further, the composition or pharmaceutical preparation of the present invention comprises 1-1500 mg of the compound, or the stereoisomer or pharmaceutically acceptable salt thereof according to any one of the preceding solutions, and a pharmaceutically acceptable carrier and/or excipient.

The present invention further provides the use of the compound, or the stereoisomer or pharmaceutically acceptable salt thereof according to any one of the preceding solutions in the preparation of a drug for the treatment/-prevention of PDE3/4-mediated diseases. Further, the PDE3/4-mediated diseases are COPD and asthma.

The present invention further provides a method for treating a disease in a mammal, comprising administering to a subject a therapeutically effective amount of the compound, or the stereoisomer or pharmaceutically acceptable salt thereof according to any one of the preceding solutions, wherein the disease is preferably COPD and asthma, and the therapeutically effective amount is preferably 1-1500 mg. In some embodiments, the mammal according to the present invention comprises humans.

The term "effective amount" or "therapeutically effective amount" according to the present application refers to a sufficient amount of the compound disclosed in the present application that is administered to ameliorate, to some extent, one or more symptoms of a disease or condition being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, and 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, and 20-30 mg; 50-1500 mg, 50-1000 mg, 50-900 mg, 50-800 mg, 50-700 mg, 50-600 mg, 50-500 mg, 50-400 mg, 50-300 mg, 50-250

mg, 50-200 mg, 50-150 mg, 50-125 mg, and 50-100 mg; and 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

In some embodiments, the pharmaceutical composition or preparation of the present invention contains the above-mentioned therapeutically effective amount of the compound, or the stereoisomer, solvate, or pharmaceutically acceptable salt thereof of the present invention.

The present invention relates to a pharmaceutical composition or pharmaceutical preparation comprising a therapeutically effective amount of the compound, or the stereoisomer or pharmaceutically acceptable salt thereof according to the present invention, and a carrier and/or excipient. The pharmaceutical composition may be in a unit preparation form (the amount of the active drug in the unit preparation is also referred to as the "preparation strength"). In some embodiments, the pharmaceutical composition comprises, but is not limited to 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, or 1500 mg of the compound, or the stereoisomer or pharmaceutically acceptable salt thereof of the invention.

The method for treating a disease in a mammal comprises administering to a subject a therapeutically effective amount of the compound, or the stereoisomer or pharmaceutically acceptable salt thereof according to the present invention, and a pharmaceutically acceptable carrier and/or excipient, wherein the therapeutically effective amount is preferably 1-1500 mg, and the disease is preferably COPD and asthma.

The method for treating a disease in a mammal comprises administering to a subject a drug, i.e., the compound, or the stereoisomer or pharmaceutically acceptable salt thereof according to the present invention, and a pharmaceutically acceptable carrier and/or excipient in a daily dose of 1-1500 mg/day, wherein the daily dose may be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; and in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1500 mg/day.

The present invention relates to a kit, which may comprise a composition in a single-dose or multiple-dose form, wherein the kit comprises the compound, or the stereoisomer or pharmaceutically acceptable salt thereof according to the present invention, and the amount of the compound, or the stereoisomer or pharmaceutically acceptable salt thereof according to the present invention is the same as that in the above-mentioned pharmaceutical composition.

In the present invention, the amount of the compound, or the stereoisomer or pharmaceutically acceptable salt thereof according to the present invention is calculated in the form of a free base in each case.

The term "preparation strength" refers to the weight of the active drug contained in each vial, tablet or other unit preparation.

## Synthetic Route

Those skilled in the art would have been able to prepare the compounds of the present invention according to known organic synthesis techniques, and the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992)

John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalog are optionally prepared by custom chemical synthesis plants, wherein many of standard chemical supply plants (such as those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

**Term**

Unless otherwise specified, the terms of the present invention have the following meanings.

The carbon, hydrogen, oxygen, sulfur, nitrogen and halogen involved in the groups and compounds of the present invention all comprise isotopes thereof, and are optionally further substituted with one or more of the corresponding isotopes thereof, wherein the isotopes of carbon comprise $^{12}C$, $^{13}C$ and $^{14}C$; the isotopes of hydrogen comprise protium (H), deuterium (deuterium, also known as heavy hydrogen), and tritium (T, also known as superheavy hydrogen); the isotopes of oxygen comprise $^{16}O$, $^{17}O$ and $^{18}O$; the isotopes of sulfur comprise $^{32}S$, $^{33}S$, $^{34}S$ and $^{36}S$; the isotopes of nitrogen comprise $^{14}N$ and $^{15}N$; the isotope of fluorine comprises $^{19}F$; the isotopes of chlorine comprise $^{35}Cl$ and $^{37}Cl$; and the isotopes of bromine comprise $^{79}Br$ and $^{81}Br$.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H), which is synonymous with "D".

The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "$C_{x-y}$" refers to a group comprising x to y carbon atoms, for example, "$C_{1-6}$ alkyl" refers to an alkyl comprising 1-6 carbon atoms.

The term "alkyl" refers to a monovalent linear or branched saturated aliphatic hydrocarbon group, usually an alkyl of 1 to 20 carbon atoms, or an alkyl of 1 to 8 carbon atoms, or an alkyl of 1 to 6 carbon atoms, or an alkyl of 1 to 4 carbon atoms. Non-limiting examples of alkyl include methyl, ethyl, n-propyl, isopropyl, n-butyl, sec-butyl, neobutyl, tert-butyl, n-pentyl, isopentyl, neopentyl, n-hexyl, etc., and alkyl may be further substituted with a substituent.

The term "alkylene" refers to a divalent linear or branched saturated alkyl. Examples of alkylene include, but are not limited to methylene, ethylidene, etc.

The term "haloalkyl" refers to an alkyl in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl. Without particular limitation, the number of halogen substituents is any integer between 1 and the upper limit. Generally, the alkyl is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to - $CF_3$, $-CH_2Cl$, $-CH_2CF_3$, $-CCl_2$, $CF_3$, etc.

The term "alkoxy" or "alkyloxy" refers to -O-alkyl, such as -O-$C_{1-8}$alkyl, -O-$C_{1-6}$alkyl, -O-$C_{1-4}$alkyl or -O-$C_{1-2}$alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo$C_{1-8}$alkyl, -O-halo$C_{1-6}$alkyl, -O-halo$C_{1-4}$alkyl or -O-halo$C_{1-2}$alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, etc.

The term "alkenyl" refers to a linear or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a linear or branched divalent unsaturated hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Non-limiting examples of alkenylene include ethynylene and the alkenylene may be optionally substituted with a substituent.

The term "alkynyl" refers to a linear or branched hydrocarbon group comprising at least one carbon-carbon triple bond (C=C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a linear or branched divalent unsaturated hydrocarbon group containing a carbon-carbon triple bond (C=C) and generally comprises 2 to 18 carbon atoms, further comprises 2 to 8 carbon atoms, further comprises 2 to 6 carbon atoms, and further comprises 2 to 4 carbon atoms. Non-limiting examples of alkynylene include ethynylene, propynylene and butynylene; and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aromatic rings, but the ring system is non-aromatic as a whole, and the attachment site may be on an aromatic ring or a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

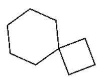, 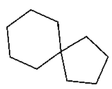, 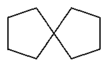,

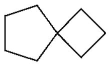, 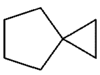, 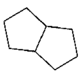, 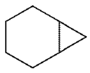, 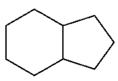, 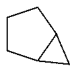, 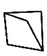, 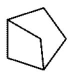, 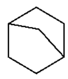, 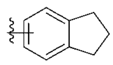,

etc., and the cycloalkyl may be optionally substituted with a substituent.

The term "cycloalkylene" refers to a divalent group of cycloalkyl.

The term "aryl" refers to an aromatic carbocycle that does not contain a heteroatom, including monocyclic aryl and fused aryl. Generally, the aryl contains 6 to 14 carbon atoms, and further contains 6 to 10 carbon atoms. Non-limiting examples of aryl include phenyl, naphthyl, anthryl, phenanthryl, and the aryl may be optionally substituted with a substituent.

"Carbocycle" or "carbocyclyl" refers to a saturated, partially unsaturated, or aromatic carbocycle, and its meaning includes aryl and cycloalkyl. The carbocycle may be monocyclic, bicyclic or polycyclic, and the bicyclic or polycyclic carbocycle may be in the form of a bridged ring, a fused ring, a spiro ring and a combination thereof. Generally, the carbocycle contains 3-12 carbon atoms, or 3-10 carbon atoms, or 3-6 carbon atoms. In a non-limiting example, the monocyclic carbocycle includes cyclopropyl, cyclobutyl, cyclopentyl, cyclohexyl, cycloheptyl, phenyl, *etc.,* the bicyclic bridged ring includes

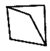, 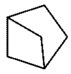, 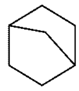,

etc., the bicyclic fused ring includes

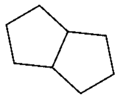, 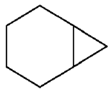, 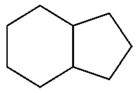, 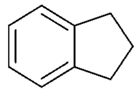, 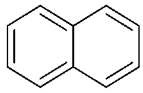, 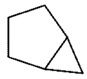,

etc., and the bicyclic spiro ring includes

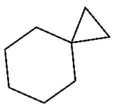, 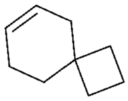, 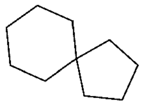,

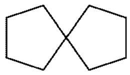, 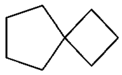, 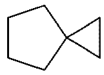,

etc. The carbocycle may be optionally substituted with a substituent.

"Heterocycloalkyl" refers to a saturated or partially unsaturated non-aromatic carbocycle containing 1, 2, 3, or 4 heteroatoms selected from N, S and O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole, and the attachment site may be on an aromatic ring or a non-aromatic ring. Generally, the heterocycloalkyl is a 3- to 20-membered ring. When the heterocycloalkyl is monocyclic heterocycloalkyl, the heterocycloalkyl is generally a 3- to 15-membered ring, or a 3- to 10-membered ring, or a 3- to 8-membered ring, or a 3- to 6-membered ring; and when the heterocycloalkyl is bicyclic or polycyclic heterocycloalkyl, the heterocycloalkyl is generally a 5- to 12-membered ring, or a 5- to 11-membered ring, or a 6- to 9-membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the attachment site. Non-limiting examples of heteroaryl ring or heteroaryl include furyl, thienyl, pyrrolyl, oxazolyl, thiazolyl, imidazolyl, pyrazolyl, pyridyl, pyrimidinyl, pyridazinyl, pyrazinyl, indolyl, purinyl,

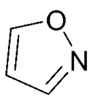, 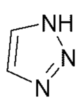, 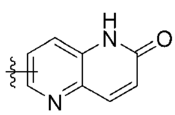,

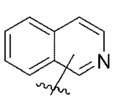, 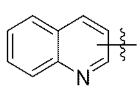, 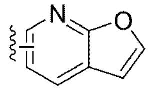, 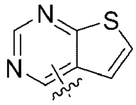, 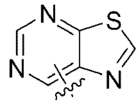, 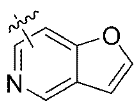,

etc. The heteroaryl may be optionally substituted with a substituent.

The term "heterocycle " or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. The heterocycle may be a monocyclic heterocycle, a bicyclic bridged heterocycle, a bicyclic fused heterocycle, a bicyclic spiro heterocycle or a combination thereof. The heterocycle is usually a 3- to 12-membered

heterocycle, or a 5- to 12-membered heterocycle, or a 5- to 7-membered heterocycle. Heterocyclyl can be attached to a heteroatom or a carbon atom. Non-limiting examples of heterocyclyl include oxiranyl, azacyclopropyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl,

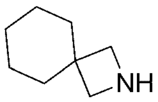, 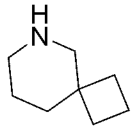, 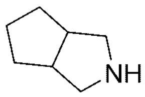, 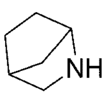, 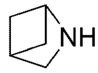,

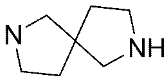, 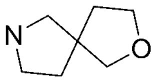, 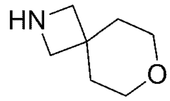, 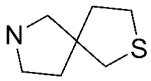,

etc. The heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include

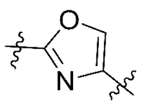, 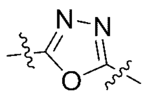, 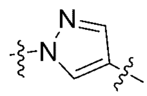, 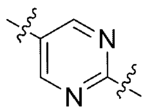,

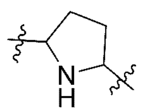,

etc.

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of the spiro ring include:

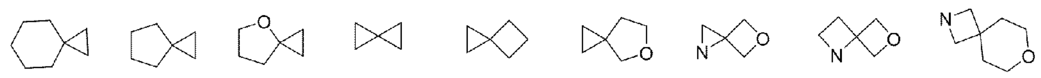

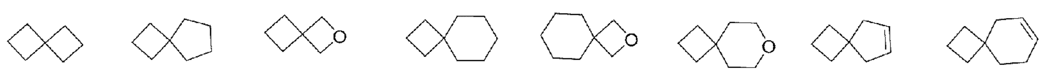

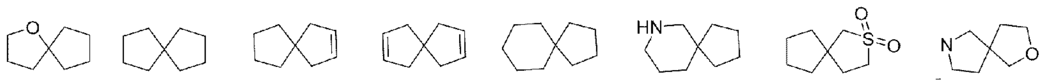

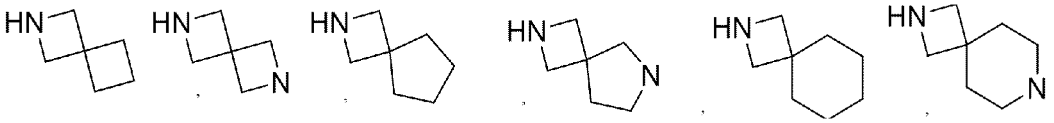

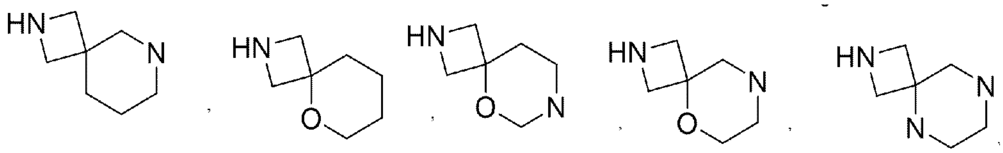

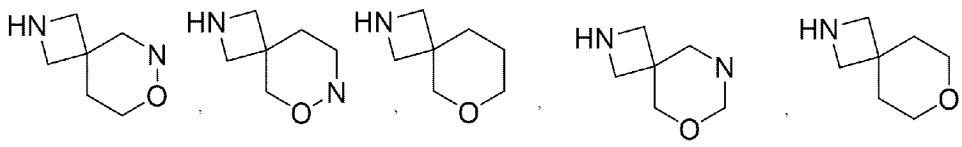

,

and the spiro ring may be optionally substituted with a substituent.

The term "fused ring (并环/稠环)" refers to a polycyclic group in which the rings share two adjacent ring atoms and one chemical bond. The fused ring may contain one or more double or triple bonds, and may contain 0 to 5 heteroatoms selected from N, S, O, P, Si and their oxidation states. Generally, the fused ring is a 5- to 20-membered ring, or a 5- to 14-membered ring, or a 5- to 12-membered ring, or a 5- to 10-membered ring. Generally, the fused ring is in the form of a three-membered ring fused with a four-membered ring (indicating a fused ring formed by a three-membered ring and a four-membered ring, wherein either the three-membered ring or the four-membered ring may be possibly used as the basic ring according to the IUPC nomenclature; similarly hereinafter), a three-membered ring fused with a five-membered ring, a three-membered ring fused with a six-membered ring, a four-membered ring fused with a four-membered ring, a four-membered ring fused with a five-membered ring, a four-membered ring fused with a six-membered ring, a five-membered ring fused with a five-membered ring, a five-membered ring fused with a six-membered ring, and a six-membered ring fused with a six-membered ring. Non-limiting examples of the fused ring include purine, quinoline, isoquinoline, benzopyran, benzofuran, benzothiophene,

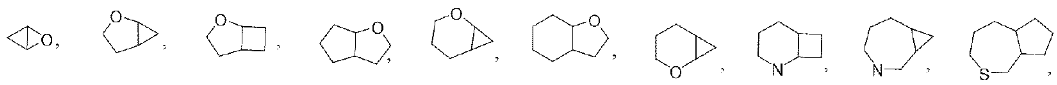

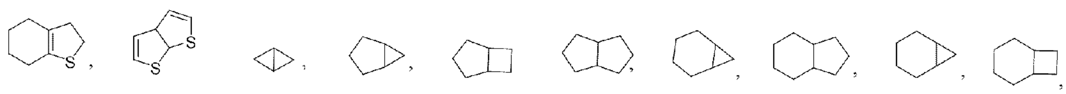

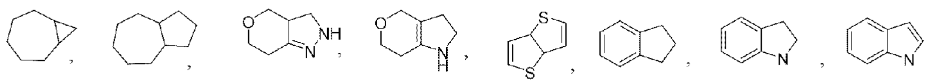

;

and the fused ring may be aromatic or non-aromatic and is optionally substituted with a substituent.

The term "bridged ring" refers to a ring system in which two non-adjacent ring atoms are shared between two rings, which may contain one or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of the bridged ring include adamantane,

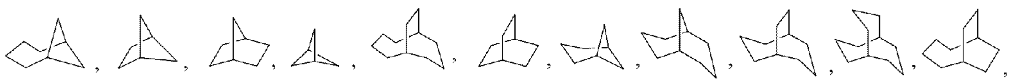

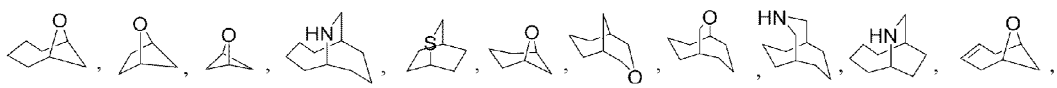

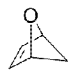, 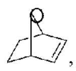, 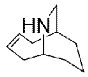, 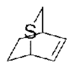, 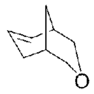, 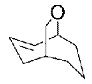, 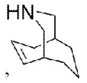, 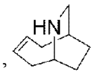, 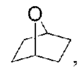, 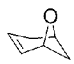

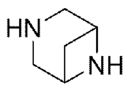, 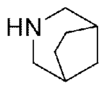, 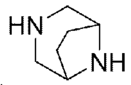, 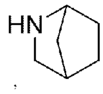, 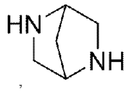, 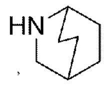, 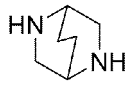,

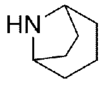, 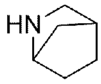, 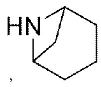, 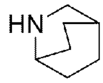, 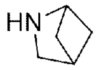, 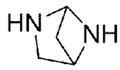, 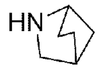

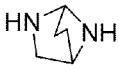, 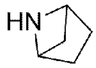, 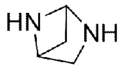, 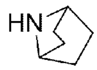

Unless otherwise specified, the term "substitution" or "substituent" refers to any substitution at a position allowed by chemical theory, and the number of substituents conforms to the rules of chemical bonding. Exemplary substituents include, but are not limited to: $C_{1-6}$alkyl, $C_{2-6}$alkenyl, $C_{2-6}$alkynyl, $C_{3-8}$heteroalkyl, $C_{5-12}$aryl, 5- to 12-membered heteroaryl, hydroxyl, $C_{1-6}$alkoxy, $C_{5-12}$aryloxy, thiol, $C_{1-6}$alkylthio, cyano, halogen, $C_{1-6}$alkylthiocarbonyl, $C_{1-6}$alkylcarbamoyl, N-carbamoyl, nitro, silyl, sulfinyl, sulfonyl, sulfoxide, halogenated $C_{1-6}$alkyl, halogenated $C_{1-6}$alkoxy, amino, phosphonic acid, $-CO_2(C_{1-6}$alkyl), -OC(=O)($C_{1-6}$alkyl), $-OCO_2(C_{1-6}$alkyl), $-C(=O)NH_2$, -C(=O)N($C_{1-6}$alkyl)$_2$, -OC(=O)NH($C_{1-6}$alkyl), -NHC(=O)($C_{1-6}$alkyl), -N($C_{1-6}$alkyl)C(=O)($C_{1-6}$alkyl), $-NHCO_2(C_{1-6}$alkyl), - NHC(=O)N($C_{1-6}$alkyl)$_2$, -HC(=O)NH($C_{1-6}$alkyl), $-NHC(=O)NH_2$, $-NHSO_2(C_{1-6}$alkyl), $-SO_2N(C_{1-6}$alkyl)$_2$, $-SO_2NH(C_{1-6}$alkyl), $-SO_2NH_2$, $-SO_2C_{1-6}$alkyl, etc.

The term "optional" or "optionally" refers to that the events or circumstances subsequently described may or may not necessarily occur, and the description includes the occasions where the events or circumstances occur or do not occur. For example, "alkyl optionally substituted with F" means that the alkyl may or may not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, solvates, pharmaceutically acceptable salts or co-crystals thereof and other components comprising physiologically/pharmaceutically acceptable carriers and/or excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavoring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatin; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerin, sorbitol, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compound of the present invention also includes the tautomers thereof, for example, when the present invention describes the left side compound in which the pyrimidine ring is substituted with OH, the right side tautomer compound is also included.

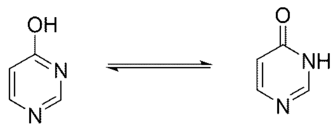

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

**Detailed Description of Embodiments**

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

**Example 1:** (*E*)-N-(2-(9-(benzyloxy)-2-(mesitylimino)-10-methoxy-4-oxo-6,7-dihydro-2H-pyrimido[6,1-a]isoquinolin-3(4H)-yl)ethyl)-4-hydroxy-1-methyl-1H-1,2,3-triazole-5-carboxamide **(Compound 1)**

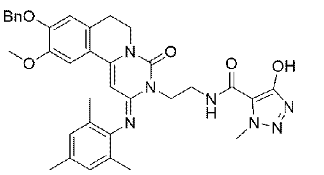

Compound 1

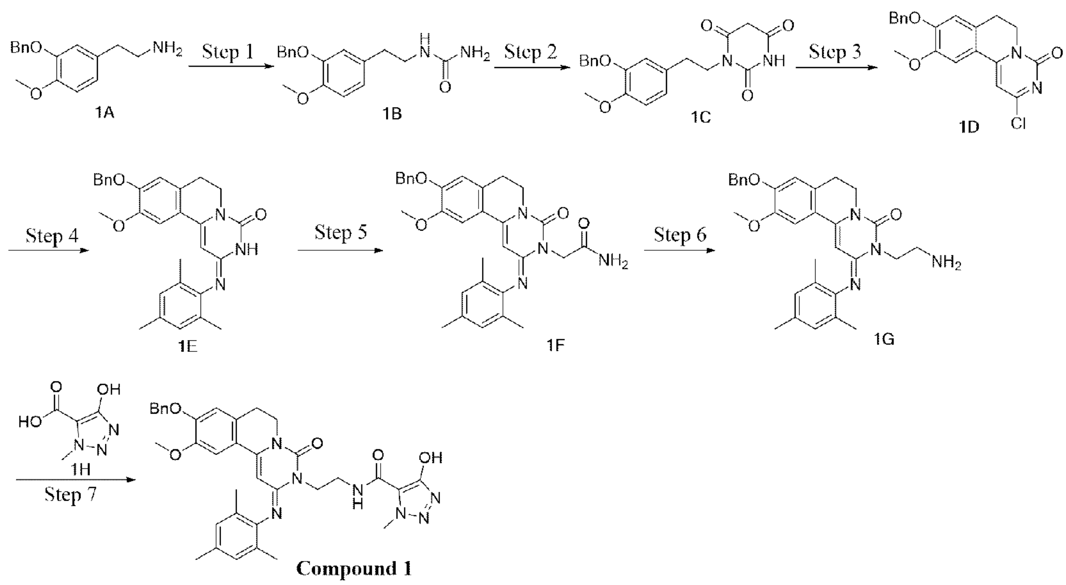


1A 1B 1C 1D 1E 1F 1G 1H

Compound 1

Step 1:

Compound 2-(3-(benzyloxy)-4-methoxyphenyl)ethan-1-amine **(1A)** (synthesized with reference to J. Med. Chem. 2013, 56, 23, 9673-9682) (51.5 g, 0.20 mol) was dissolved in water (300 mL), potassium cyanate (32.4 g, 0.40 mol) was added portionwise, the mixture was stirred until uniform, and then 2 N hydrochloric acid (240 mL) was added dropwise. After the dropwise addition was completed, the mixture was refluxed and reacted overnight. The reaction solution was cooled to 0°C and filtered, and the filter cake was rinsed with ice ethanol and dried, to afford the target compound **1B** (38.0 g, yield: 63%).

LC-MS (ESI): m/z = 301.2 $[M+H]^+$.

Step 2:

Compound **1B** (38 g, 0.13 mol) was dissolved in anhydrous ethanol (300 mL), diethyl malonate (27 g, 0.17 mol) was added, the mixture was stirred until uniform, and then sodium ethoxide (133 g, 0.39 mol, 20 wt% in EtOH) was added dropwise. After the dropwise addition was completed, the mixture was refluxed and reacted overnight. The reaction solution was cooled to room temperature, concentrated to 150 mL under reduced pressure, diluted with water (150 mL), adjusted to pH 6.0 with 5 N hydrochloric acid and filtered, and the filter cake was washed with water (150 mL) and dried, to afford the target compound **1C** (42 g, yield: 90%).

LC-MS (ESI): m/z = 369.1 $[M+H]^+$.

Step 3:

Compound **1C** (42 g, 114 mmol) was dissolved in phosphorus oxychloride (300 mL), and the mixture was reacted overnight at 120°C. After the reaction was completed, the reaction solution was cooled to room temperature and concentrated under reduced pressure, the residue was slowly added to ice water (300 mL), and saturated sodium bicarbonate aqueous solution was added to adjust to pH = 6.0. The mixture was extracted with dichloromethane (500 mL × 3), the organic phases were combined, dried over anhydrous sodium sulfate and filtered, the filtrate was concentrated under reduced pressure, and the resulting crude product was separated by silica gel column chromatography (DCM : MeOH (v/v) = 40 : 1), to afford the target compound **1D** (12.7 g, yield: 30%).

LC-MS (ESI): m/z = 369.1 $[M+H]^+$.

$^1H$ NMR (400 MHz, $CDCl_3$) δ 7.48-7.32 (m, 5H), 7.14 (s,1H), 6.80 (s, 1H), 6.64 (s, 1H), 5.23 (s, 2H), 4.20 (t, 2H), 3.96 (s, 3H), 2.94 (t, 2H).

Step 4:

**1D** (8.2 g, 22.2 mmol) was dissolved in isopropanol (120 mL), 2,4,6-trimethylaniline (4.46 g, 33 mmol) was added, and the mixture was reacted at 90°C for 24 hours after the addition was completed. After the reaction was completed, the reaction solution was cooled to room temperature and concentrated under reduced pressure, saturated sodium bicarbonate aqueous solution (100 mL) was added to the residue, and the mixture was extracted with dichloromethane (100 mL× 3). The organic phases were combined, washed with saturated sodium chloride aqueous solution (100 mL), dried over anhydrous sodium sulfate and filtered, the filtrate was concentrated under reduced pressure, and the residue was separated by silica gel column chromatography (DCM : MeOH (v/v) = 20 : 1), to afford **compound 1E** (8.3 g, yield: 80%).

LC-MS (ESI): m/z = 468.2 $[M+H]^+$.

Step 5:

**Compound 1E** (1.2 g, 2.6 mmol), 2-bromoacetamide (1.08 g, 7.8 mmol), potassium phosphate (2.76 g, 13 mmol) and sodium iodide (1.17 g, 7.8 mmol) were successively dissolved in dry 2-butanone (40 mL), and the mixture was reacted under nitrogen atmosphere at 90°C for 18 hours. After the reaction was completed, the reaction solution was cooled to room temperature and filtered, the filter cake was washed with dichloromethane (80 mL), the filtrate was concentrated under reduced pressure, and the resulting crude product was separated by silica gel column chromatography (dichloromethane: methanol (v/v) = 1 : 0-20 : 1), to afford the target compound **IF** (1.1 g, yield: 82%).

LC-MS (ESI): m/z = 525.3 $[M+H]^+$.

Step 6:

Borane dimethyl sulfide (2.1 mL, 10 N) was added to tetrahydrofuran (20 mL), and **compound 1F** (1.1 g, 2.1

mmol) was added portionwise at 0°C, and then the mixture was reacted overnight at room temperature after the addition was completed. After the reaction was completed, methanol (10 mL) was slowly added dropwise to quench the reaction, and 2 N hydrochloric acid (30 mL) was added dropwise after no bubbles were generated, and the mixture was stirred for another 10 minutes. The reaction solution was concentrated under reduced pressure, saturated sodium bicarbonate aqueous solution (30 mL) was added to the residue, and the mixture was extracted with dichloromethane (50 mL × 3). The organic phases were combined, washed with saturated sodium chloride aqueous solution (100 mL), dried over anhydrous sodium sulfate and filtered, the filtrate was concentrated under reduced pressure, and the residue was separated by silica gel column chromatography (DCM : MeOH (v/v) = 8 : 1), to afford **compound 1G** (0.12 g, yield: 11%).

LC-MS (ESI): m/z = 511.2 $[M+H]^+$.

Step 7:

**1H** (synthesized with reference to the method described in patent WO 2020011254) (33 mg, 0.23 mmol) was dissolved in DMF (5 mL) and then HATU (89 mg, 0.23 mmol) and DIPEA (65 mg, 0.5 mmol) were added, followed by stirring for 10 minutes, and then a solution of **compound 1G** (0.12 g, 0.23 mmol) in DMF (2 mL) was added dropwise, and the mixture was reacted at room temperature for 1 hour after the dropwise addition was completed. After the reaction was completed, saturated sodium chloride aqueous solution (50 mL) was added, and the mixture was extracted with dichloromethane (50 mL × 2). The organic phases were combined, washed with saturated sodium chloride aqueous solution (60 mL), dried over anhydrous sodium sulfate, filtered and concentrated, and the residue was separated by silica gel column chromatography (DCM : MeOH(v/v) = 15 : 1), to afford the **compound 1** (105 mg, yield: 70%).

LC-MS (ESI): m/z = 636.3 $[M+H]^+$.

$^1H$ NMR (400 MHz, $CDCl_3$) δ 7.43-7.28 (m, 5H), 6.86 (s, 2H), 6.74 (s, 1H), 6.69 (s, 1H), 5.48 (s, 1H), 5.15 (s, 2H), 4.59 (s, 2H), 4.22 (s, 3H), 4.04 (t, 2H), 3.89 (s, 2H), 3.76 (s, 3H), 2.83 (t, 2H), 2.26 (s, 3H), 2.04 (s, 6H).

**Example 2:** (*E*)-4-hydroxy-N-(2-(2-(mesitylimino)-9,10-dimethoxy-4-oxo-6,7-dihydro-2H-pyrimido[6,1-a]isoquinolin-3(4H)-yl)ethyl)-1-methyl-1H-1,2,3-triazole-5-carboxamide **(Compound 2)**

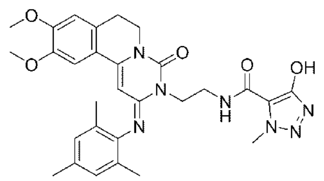

**Compound 2**

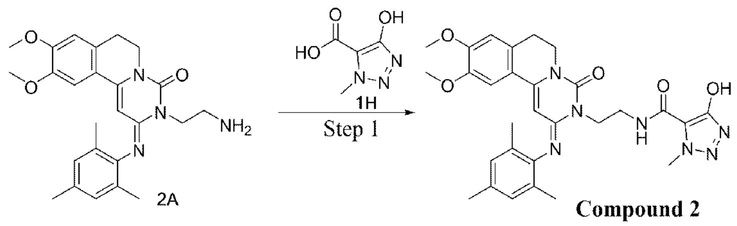


2A

**Compound 2**

Step 1:

**1H** (43 mg, 0.3 mmol) was dissolved in DMF (5 mL) and then HATU (0.11 g, 0.3 mmol) and DIPEA (40 mg, 0.3 mmol) were successively added, followed by stirring for 10 minutes, and then a solution of **compound 2A** (0.12 g, 0.28 mmol) (prepared with reference to the method described in patent WO 00058308) in DMF (2 mL) was added dropwise, and the mixture was reacted at room temperature for 1 hour after the dropwise addition was completed. After the reaction was completed, saturated sodium chloride aqueous solution (50 mL) was added, and the mixture was extracted with dichloromethane (50 mL × 2). The organic phases were combined, washed with saturated sodium chloride aqueous solution (60 mL), dried over anhydrous sodium sulfate, filtered and concentrated, and the residue was separated by silica gel column chromatography (DCM : MeOH(v/v) = 15 : 1), to afford the **compound 2** (0.12 g, yield: 78%).

LC-MS (ESI): m/z = 560.3 $[M+H]^+$.

$^1H$ NMR (400 MHz, $CDCl_3$) δ 6.86 (s, 2H), 6.70 (s, 1H), 6.66 (s, 1H), 5.48 (s, 1H), 4.63 (s, 2H), 4.23 (s, 3H), 4.08 (t,

2H), 3.90 (s, 5H), 3.75 (s, 3H), 2.90 (t, 2H), 2.26 (s, 3H), 2.05 (s, 6H).

**Example 3:** (E)-N-(2-(9-(cyclopropylmethoxy)-2-(mesitylimino)-10-methoxy-4-oxo-6,7-dihydro-2H-pyrimido[6,1-a]isoquinolin-3(4H)-yl)ethyl)-4-hydroxy-1-methyl-1H-1,2,3-triazole-5-carboxamide **(Compound 3)**

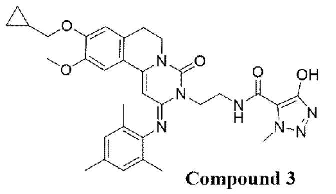

Compound 3

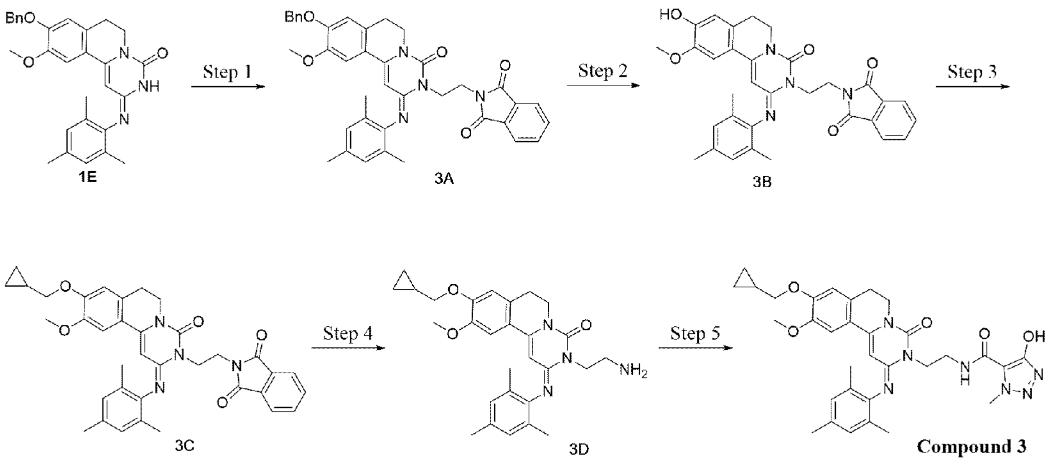


1E 3A 3B

3C 3D Compound 3

Step 1:

Compound **1E** (8.14 g, 17.41 mmol) was dissolved in 2-butanone (200 mL) and then N-(2-bromoethyl) phthalimide (26.54 g, 104.46 mmol), potassium carbonate (21.66 g, 156.7 mmol) and sodium iodide (15.66 g, 104.46 mmol) were successively added, and the mixture was heated to 90°C and refluxed for 48 hours. TLC/LCMS showed that most of the raw materials did not react completely, and some products appeared. After the mixture was cooled to room temperature and filtered, the filter cake was washed twice with dichloromethane (40 mL), and the filtrate was concentrated under reduced pressure. The resulting crude product was purified by silica gel column chromatography (petroleum ether : ethyl acetate = 5 : 1 - dichloromethane:methanol = 20 : 1), to afford compound **3A** (1.54 g, yield: 14%).

LC-MS (ESI): m/z = 641.2 $[M+H]^+$.

Step 2:

Compound **3A** (1.34 g, 2.09 mmol) was dissolved in ethyl acetate (50 mL), palladium on carbon (0.3 g, 10% w/w) was added, and the mixture was reacted under hydrogen atmosphere at room temperature for 3 hours. After raw materials were completely reacted as monitored by TLC/LCMS, the reaction solution was filtered to remove palladium on carbon, the filter cake was washed twice with ethyl acetate (20 mL), the filtrate was concentrated under reduced pressure, and the resulting crude product was purified by silica gel column chromatography (petroleum ether : ethyl acetate = 3 : 1), to afford compound **3B** (0.78 g, yield: 68%).

LC-MS (ESI): m/z = 551.2 $[M+H]^+$.

Step 3:

Compound **3B** (350 mg, 0.64 mmol) was dissolved in DMF (20 mL), (bromomethyl)cyclopropane (173 mg, 1.28 mmol) and potassium carbonate (177 mg, 1.28 mmol) were successively added, and the mixture was stirred at room temperature for 5 hours. After raw materials were completely reacted as monitored by TLC/LCMS, the reaction was quenched with saturated sodium chloride aqueous solution (30 mL), and the mixture was extracted with dichloromethane

(40 mL × 2). The organic phases were combined, dried over anhydrous sodium sulfate, filtered and concentrated under reduced pressure, and the resulting crude product was purified by silica gel column chromatography (petroleum ether : ethyl acetate = 3 : 1), to afford compound **3C** (220 mg, yield: 57%).

LC-MS (ESI): m/z = 605.3 $[M+H]^+$.

Step 4:

Compound **3C** (220 g, 0.36 mmol) was dissolved in chloroform (10 mL) and ethanol (10 mL), hydrazine hydrate (338 mg, 5.4 mmol) was added, and the mixture was stirred overnight at room temperature after the addition was completed. After the reaction was completed as monitored by TLC/LCMS, the reaction was quenched with saturated sodium bicarbonate aqueous solution (20 mL), and the mixture was extracted with dichloromethane (30 mL × 2). The organic phases were combined, dried over anhydrous sodium sulfate, filtered and concentrated under reduced pressure to afford compound **3D** (123 mg, yield: 72%).

LC-MS (ESI): m/z = 475.2 $[M+H]^+$.

Step 5:

4-hydroxy-1-methyl-1H-1,2,3-triazole-5-carboxylic acid **(1H)** (18.6 mg, 0.13 mmol) was dissolved in DMF (10 mL), then DIPEA (50.4 mg, 0.39 mmol) and HATU (74.1 mg, 0.2 mmol) were successively added, followed by stirring for 10 minutes, then compound **3D** (61.7 mg, 0.13 mmol) was added, and the mixture was reacted at room temperature for 3 hours. After the reaction was completed as monitored by TLC/LCMS, the reaction was quenched with saturated sodium chloride aqueous solution (20 mL), and the mixture was extracted with dichloromethane (30 mL × 2). The organic phases were combined, dried over anhydrous sodium sulfate, filtered and concentrated under reduced pressure, and the resulting crude product was purified by silica gel column chromatography (dichloromethane : methanol = 20 : 1), to afford the **compound 3** (20 mg, yield: 26%).

LC-MS (ESI): m/z = 600.3 $[M+H]^+$.

$^1$H NMR (400 MHz, Chloroform-d) δ 6.87 (s, 2H), 6.71 (s, 1H), 6.64 (s, 1H), 5.48 (s, 1H), 4.63 (m, 2H), 4.23 (s, 3H), 4.08-4.07 (m, 2H), 3.91-3.90 (m, 2H), 3.88-3.86 (m, 2H), 3.75 (s, 3H), 2.88-2.87 (m, 2H), 2.26 (s, 3H), 2.05 (s, 6H), 1.35-1.34 (m, 1H), 0.66-0.65 (m, 2H), 0.36-0.34 (m, 2H).

**Example 4:** (*E*)-4-hydroxy-N-(2-(2-(mesitylimino)-10-methoxy-4-oxo-9-(2,2,2-trifluoroethoxy)-6,7-dihydro-2H-pyrimido[6,1-a]isoquinolin-3(4H)-yl)ethyl)-1-methyl-1H-1,2,3-triazole-5-carboxamide **(Compound 4)**

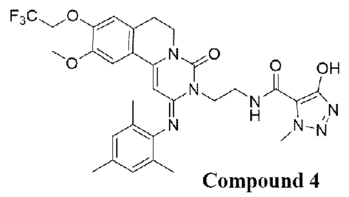

Compound 4

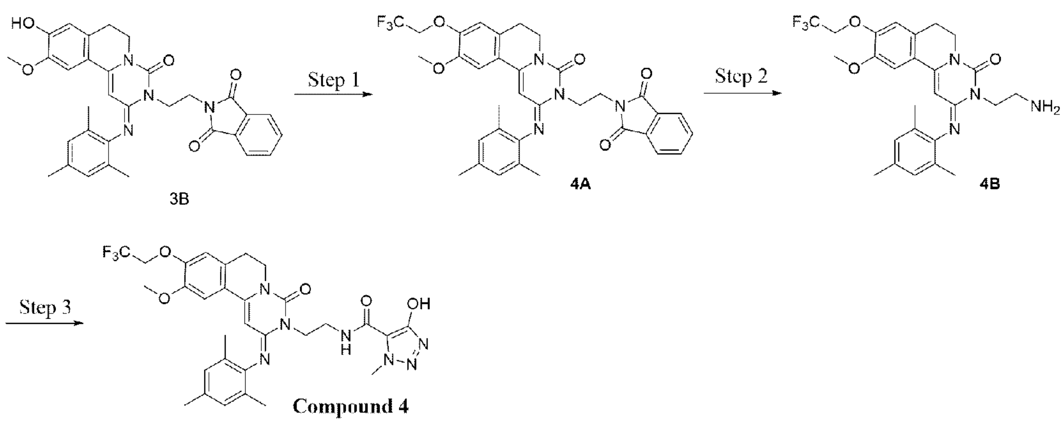


3B 4A 4B

Compound 4

Step 1:

Compound **3B** (430 mg, 0.78 mmol) was dissolved in DMF (10 mL), then 2,2,2-trifluoroethyl trifluoromethanesulfonate (362 mg, 1.56 mmol) and potassium carbonate (216 mg, 1.56 mmol) were successively added at room temperature, and the mixture was reacted at room temperature for 3 hours after the addition was completed. After the reaction was completed as monitored by TLC/LCMS, the reaction was quenched with saturated sodium chloride aqueous solution (20 mL), and the mixture was extracted with dichloromethane (30 mL × 2). The organic phases were combined, dried over anhydrous sodium sulfate, filtered and concentrated under reduced pressure, and the resulting crude product was purified by silica gel column chromatography (petroleum ether : ethyl acetate = 3 : 1), to afford compound **4A** (370 mg, yield: 75%).

LC-MS (ESI): m/z = 633.2 $[M+H]^+$.

Step 2:

Compound **4A** (370 mg, 0.58 mmol) was dissolved in chloroform (10 mL) and ethanol (10 mL), hydrazine hydrate (544 mg, 8.7 mmol) was added, and the mixture was stirred overnight at room temperature. After the reaction was completed as monitored by TLC/LCMS, the reaction was quenched with saturated sodium bicarbonate aqueous solution (10 mL), and the mixture was extracted with dichloromethane (20 mL × 2). The organic phases were combined, dried over anhydrous sodium sulfate, filtered and concentrated, to afford compound **4B** (156 mg, yield: 54%).

LC-MS (ESI): m/z = 503.2 $[M+H]^+$.

Step 3:

4-hydroxy-1-methyl-1H-1,2,3-triazole-5-carboxylic acid **(1H)** (22.9 mg, 0.16 mmol) was dissolved in DMF (10 mL), then DIPEA (62 mg, 0.48 mmol) and HATU (91.3 mg, 0.24 mmol) were successively added at room temperature, followed by stirring for 10 minutes, then compound **4B** (78 mg, 0.16 mmol) was added, and the mixture was reacted at room temperature for 3 hours. After the reaction was completed as monitored by TLC/LCMS, the reaction was quenched with saturated sodium chloride aqueous solution (20 mL), and the mixture was extracted with dichloromethane (30 mL × 2). The organic phases were combined, dried over anhydrous sodium sulfate, filtered and concentrated under reduced pressure, and the resulting crude product was purified by silica gel column chromatography (dichloromethane : methanol = 20 : 1), to afford the **compound 4** (9 mg, yield: 9%).

LC-MS (ESI): m/z = 628.2 $[M+H]^+$.

$^1$H NMR (400 MHz, Chloroform-d) δ 6.88 (s, 2H), 6.79 (s, 1H), 6.74 (s, 1H), 5.52 (s, 1H), 4.72-4.70 (m, 2H), 4.43-4.42 (m, 2H), 4.22 (s, 3H), 4.11-4.09 (m, 2H), 3.97-3.95 (m, 2H), 3.75 (s, 3H), 2.93-2.91 (m, 2H), 2.27 (s, 3H), 2.06 (s, 6H).

**Compound 5:** (*E*)-N-(2-(2-((4-fluoro-2,6-dimethylphenyl)imino)-9,10-dimethoxy-4-oxo-6,7-dihydro-2H-pyrimido[6,1-a]isoquinolin-3 (4H)-yl)ethyl)-4-hydroxy-1-methyl-1H-1,2,3-triazole-5-carboxamide **(Compound 5)**

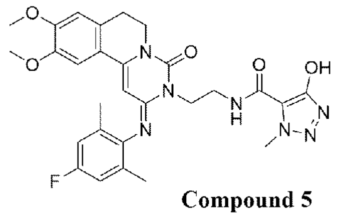

Compound 5

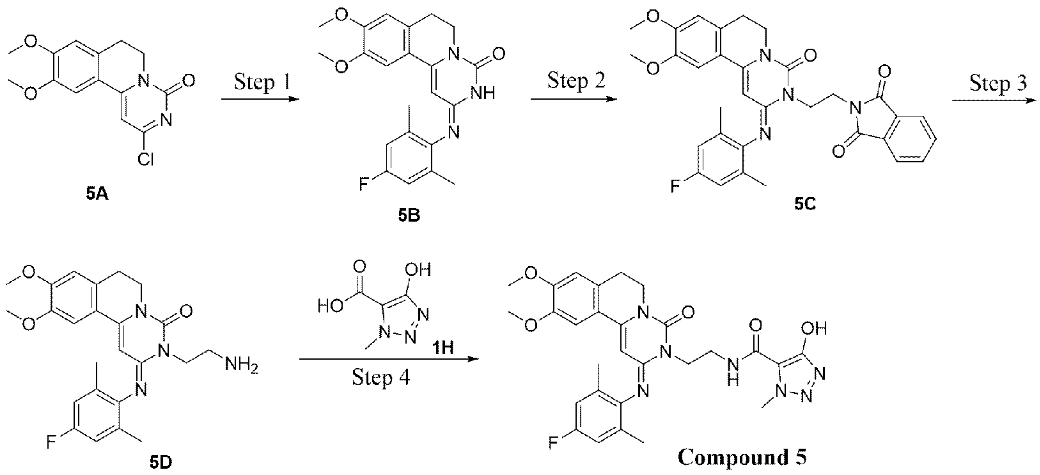


5A 5B 5C

1H

5D Compound 5

Step 1:

**5A** (0.5 g, 1.71 mmol) (prepared with reference to the method described in patent WO 00058308) was dissolved in isopropanol (12 mL), 4-fluoro-2,6-dimethylaniline (0.32 g, 2.57 mmol) was added, and the mixture was reacted at 90°C for 12 hours after the addition was completed. After the reaction was completed, the reaction solution was concentrated under reduced pressure, saturated sodium bicarbonate aqueous solution (20 mL) was added, and the mixture was extracted with dichloromethane (20 mL × 3). The organic phases were combined, washed with saturated sodium chloride aqueous solution (20 mL), dried over anhydrous sodium sulfate and filtered, the filtrate was concentrated under reduced pressure, and the resulting residue was separated by silica gel column chromatography (DCM : MeOH (v/v) = 20 : 1), to afford **compound 5B** (0.57 g, yield: 85%).
LC-MS (ESI): m/z =396.2 $[M+H]^+$.

Step 2:

**Compound 5B** (0.57 g, 1.5 mmol), 2-(2-bromoethyl)isoindolin-1,3-dione (1.15 g, 4.5 mmol), potassium carbonate (0.62 g, 4.5 mmol) and sodium iodide (0.69 g, 4.5 mmol) were successively dissolved in dry acetonitrile (20 mL), and the mixture was reacted under nitrogen protection and microwave at 120°C for 2 hours. After the reaction was completed, the reaction solution was cooled to room temperature and filtered, the filter cake was washed with dichloromethane (20 mL), and the filtrate was concentrated under reduced pressure and then separated by silica gel column chromatography (petroleum ether: ethyl acetate (v/v) = 1 : 0-1 : 1), to afford the target compound **5C** (0.34 g, yield: 40%).
LC-MS (ESI): m/z =569.2 $[M+H]^+$.

Step 3:

**Compound 5C** (0.34 g, 0.60 mmol) was dissolved in trichloromethane (10 mL) and ethanol (10 mL), hydrazine hydrate (0.30 g, 80 wt%) was added, and the mixture was stirred until uniform and then reacted at room temperature for 12 hours. After the reaction was completed, the reaction solution was filtered, the filtrate was concentrated under reduced pressure, and the residue was separated by silica gel column chromatography (dichloromethane: methanol (v/v) = 1 : 0-5 : 1), to afford **compound 5D** (0.17 g, yield: 89%).
LC-MS (ESI): m/z = 439.1 $[M+H]^+$.

Step 4:

**Compound 1H** (56 mg, 0.39 mmmol) was dissolved in N,N-dimethylformamide (4 mL), then DIPEA (151 mg, 1.17 mmol) and HATU (163 mg, 0.43 mmol) were successively added, followed by stirring at room temperature for 15 minutes, then **compound 5D** was added, and the mixture was stirred until uniform and then reacted at room temperature for 3 hours. After the reaction was completed, the reaction solution was filtered, the filtrate was concentrated under reduced pressure, and the residue was separated by silica gel column chromatography (dichloromethane: methanol (v/v) = 1 : 0-5 : 1), to afford the **compound 5** (0.07 g, yield: 62%).
$^1$H NMR (400 MHz, Methanol-$d_4$) δ 6.77 (s, 1H), 6.70-6.68 (m, 2H), 6.65 (s, 1H), 5.33 (s, 1H), 4.40 - 4.39 (m, 2H), 3.99 (s, 3H), 3.96 - 3.84 (m, 2H), 3.74-3.84 (m, 5H), 3.59 (s, 3H), 2.82-2.79 (m, 2H), 1.94 (s, 6H).
LC-MS (ESI): m/z = 564.3 $[M+H]^+$.

**Compound 6:** (*E*)-N-(2-(2-((4-cyclopropyl-2,6-dimethylphenyl)imino)-9,10-dimethoxy-4-oxo-6,7-dihydro-2H-pyrimido [6,1-a]isoquinolin-3(4H)-yl)ethyl)-4-hydroxy-1-methyl-1H-1,2,3-triazole-5-carboxamide (Compound 6)

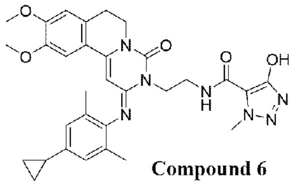

Compound 6

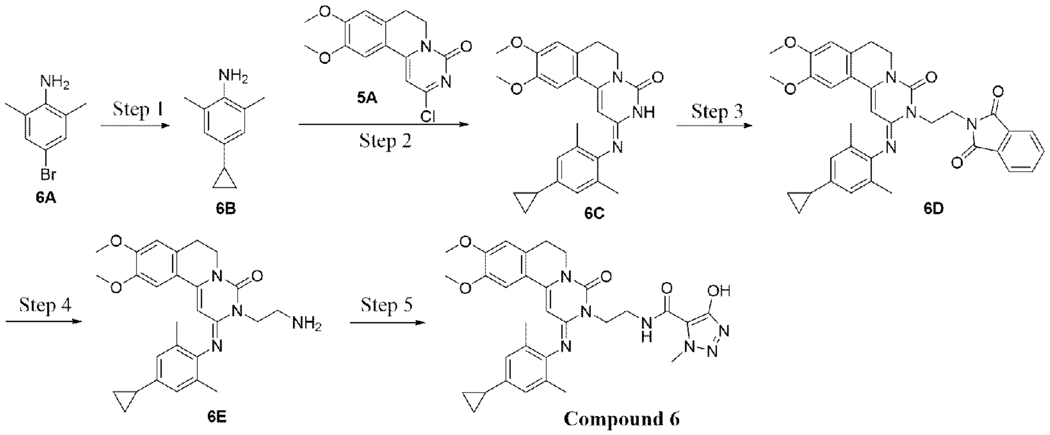


6A 6B 5A 6C 6D 6E Compound 6

Step 1:

**Compound 6A** (3 g, 15.07 mmol) and cyclopropylboronic acid (1.94 g, 22.67 mmol) were dissolved in toluene (20 mL) and water (5 mL), palladium acetate (0.34 g, 1.51 mmol), tricyclohexylphosphine (0.47 g, 28.38 mmol) and potassium phosphate (6.40 g, 30.14 mmol) were successively added, and the mixture was reacted under nitrogen atmosphere at 100°C. After the reaction was completed as monitored by TLC and LC-MS, the reaction was terminated and cooled to room temperature, most of the reaction solution was removed by concentration under reduced pressure, and DCM (20 mL) was added to the residue. The mixture was filtered over diatomaceous earth, the filtrate was concentrated under reduced pressure, and the residue was separated and purified by silica gel column (dichloromethane: methanol (v/v) = 20 : 1-10 : 1), to afford the title compound **6B** (0.70 g, yield: 29%).

LC-MS (ESI): m/z =162.2 $[M+H]^+$.

Step 2:

**5A** (0.5 g, 1.71 mmol) was dissolved in isopropanol (12 mL), **6B** (0.41 g, 2.57 mmol) was added, and the mixture was reacted at 90°C for 24 hours after the addition was completed. After the reaction was completed, the reaction solution was cooled to room temperature and concentrated under reduced pressure, saturated sodium bicarbonate aqueous solution (20 mL) was added to the residue, and the mixture was extracted with dichloromethane (20 mL × 3). The organic phases were combined, washed with saturated sodium chloride aqueous solution (20 mL), dried over anhydrous sodium sulfate, filtered and concentrated under reduced pressure, and the residue was separated by silica gel column chromatography (DCM : MeOH (v/v) = 20 : 1), to afford **compound 6C** (0.50 g, yield: 70%).

LC-MS (ESI): m/z =418.3 $[M+H]^+$.

Step 3:

**Compound 6C** (0.50 g, 1.20 mmol), 2-(2-bromoethyl)isoindolin-1,3-dione (0.92 g, 3.6 mmol), potassium carbonate (0.50 g, 3.6 mmol) and sodium iodide (0.54 g, 3.6 mmol) were successively dissolved in dry acetonitrile (20 mL), and the mixture was reacted under nitrogen protection and microwave at 120°C for 2 hours. After the reaction was completed, the reaction solution was cooled to room temperature and filtered, the filter cake was washed with dichloromethane (20 mL), and the filtrate was concentrated under reduced pressure and then separated by silica gel

column chromatography (petroleum ether: ethyl acetate (v/v) = 1 : 0-1 : 1), to afford the target compound **6D** (0.33 g, yield: 47%).

LC-MS (ESI): m/z =591.3 $[M+H]^+$.

Step 4:

**Compound 6D** (0.33 g, 0.56 mmol) was dissolved in trichloromethane (10 mL) and ethanol (10 mL), hydrazine hydrate (0.3 g, 80 wt%) was added, and the mixture was stirred until uniform and then reacted at room temperature for 24 hours. After the reaction was completed, the reaction solution was filtered, the filtrate was concentrated under reduced pressure, and the residue was separated by silica gel column chromatography (dichloromethane: methanol (v/v) = 1 : 0-5 : 1), to afford **compound 6E** (0.18 g, yield: 70%).

LC-MS (ESI): m/z = 461.2 $[M+H]^+$.

Step 5:

**Compound 1H** (56 mg, 0.39 mmol) was dissolved in N,N-dimethylformamide (4 mL), DIPEA (151 mg, 1.17 mmol) and HATU (163 mg, 0.43 mmol) were added thereto, followed by stirring for 15 minutes, then **compound 6E** was added, and the mixture was stirred until uniform and then reacted at room temperature for 3 hours. After the reaction was completed, the reaction solution was filtered, the filtrate was concentrated under reduced pressure, and the residue was separated by silica gel column chromatography (dichloromethane: methanol (v/v) = 1 : 0-5 : 1), to afford the **compound 6** (0.07 g, yield: 63%).

$^1$H NMR (400 MHz, Methanol-$d_4$) δ 6.76 (s, 1H), 6.70 (s, 2H), 6.65 (s, 1H), 5.38 (s, 1H), 4.37-4.34 (m, 2H), 4.01 (s, 3H), 3.96-3.84 (m, 2H), 3.74-3.71 (m, 5H), 3.58 (s, 3H), 2.81-2.78 (m, 2H), 1.92 (s, 6H), 1.85-1.68 (m, 1H), 0.84-0.78 (m, 2H), 0.56-0.48 (m, 2H).

LC-MS (ESI): m/z = 586.3 $[M+H]^+$.

**Compound 7:** (*E*)-N-(2-(2-((2,6-dimethylphenyl)imino)-9,10-dimethoxy-4-oxo-6,7-dihydro-2H-pyrimido[6, 1-a]isoquinolin-3(4H)-yl)ethyl)-4-hydroxy-1-methyl-1H-1,2,3-triazole-5-carboxamide **(Compound 7)**

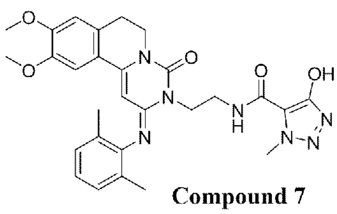

Compound 7

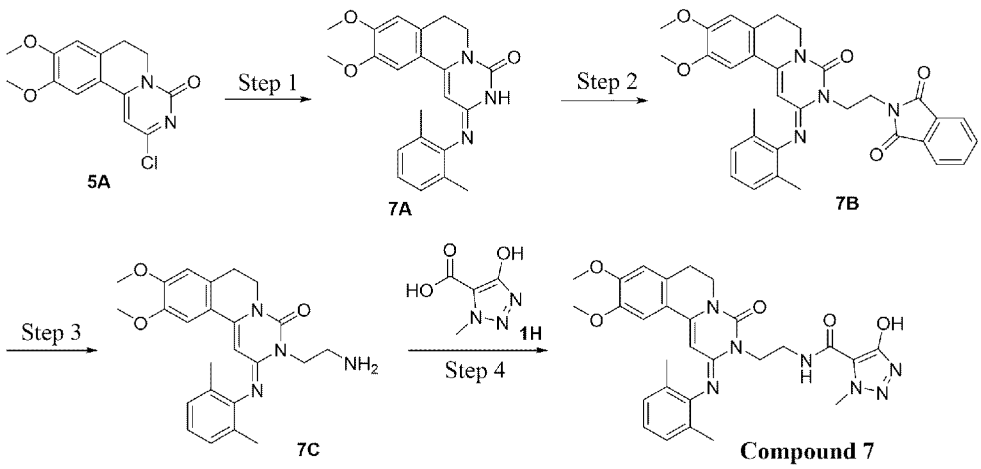


5A

7A

7B

1H

7C

Compound 7

Step 1:

**5A** (0.5 g, 1.71 mmol) was dissolved in isopropanol (12 mL), 2,6-dimethylaniline (0.32 g, 2.57 mmol) was added,

and the mixture was reacted at 90°C for 12 hours after the addition was completed. After the reaction was completed, the reaction solution was cooled to room temperature and concentrated under reduced pressure, saturated sodium bicarbonate aqueous solution (20 mL) was added to the residue, and the mixture was extracted with dichloromethane (20 mL × 3). The organic phases were combined, washed with saturated sodium chloride aqueous solution (20 mL), dried over anhydrous sodium sulfate and filtered, the filtrate was concentrated under reduced pressure, and the residue was separated by silica gel column chromatography (DCM : MeOH (v/v) = 20 : 1), to afford **compound 7A** (0.56 g, yield: 87%).
LC-MS (ESI): m/z =378.2 $[M+H]^+$.

Step 2:

**Compound 7A** (0.56 g, 1.5 mmol), 2-(2-bromoethyl)isoindolin-1,3-dione (1.15 g, 4.5 mmol), potassium carbonate (0.62 g, 4.5 mmol) and sodium iodide (0.69 g, 4.5 mmol) were successively dissolved in dry acetonitrile (20 mL), and the mixture was reacted under nitrogen protection and microwave at 120°C for 2 hours. After the reaction was completed, the reaction solution was cooled to room temperature and filtered, the filter cake was washed with dichloromethane (20 mL), and the filtrate was concentrated under reduced pressure and then separated by silica gel column chromatography (petroleum ether: ethyl acetate (v/v) = 1 : 0-1 : 1), to afford the target compound **7B** (0.34 g, yield: 41%).
LC-MS (ESI): m/z =551.2 $[M+H]^+$.

Step 3:

**Compound 7B** (0.34 g, 0.62 mmol) was dissolved in trichloromethane (10 mL) and ethanol (10 mL), hydrazine hydrate (0.30 g, 80 wt%) was added, and the mixture was stirred until uniform and then reacted at room temperature for 12 hours. After the reaction was completed, the reaction solution was filtered, the filtrate was concentrated under reduced pressure, and the residue was separated by silica gel column chromatography (dichloromethane: methanol (v/v) = 1 : 0-5 : 1), to afford **compound 7C** (0.15 g, yield: 58%).
LC-MS (ESI): m/z = 421.1 $[M+H]^+$.

Step 4:

**Compound 1H** (0.085 g, 0.20 mmol) was dissolved in N,N-dimethylformamide (4 mL), then DIPEA (0.85 g, 0.20 mmol) and HATU (0.85 g, 0.20 mmol) were successively added, followed by stirring for 15 minutes, then **compound 7C** was added, and the mixture was stirred until uniform and then reacted at room temperature for 3 hours. After the reaction was completed, the reaction solution was filtered, the filtrate was concentrated under reduced pressure, and the residue was separated by silica gel column chromatography (dichloromethane: methanol (v/v) = 1 : 0-5 : 1), to afford the **compound 7** (0.07 g, yield: 64%).
$^1H$ NMR (400 MHz, Methanol-$d_4$) δ 6.95-6.92 (m, 1H), 6.77-6.74 (m, 2H), 6.63 (s, 1H), 5.35 (s, 1H), 4.39-4.35 (m, 2H), 3.99 (s, 3H), 3.94-3.79 (m, 2H), 3.77-3.70 (m, 5H), 3.56 (s, 3H), 2.82-2.79 (m, 2H), 1.95 (s, 6H).
LC-MS (ESI): m/z = 546.3 $[M+H]^+$.

**Example 8:** (*E*)-N-(2-(2-((4-cyclopropyl-2,6-dimethylphenyl)imino)-9-ethoxy-10-methoxy-4-oxo-6,7-dihydro-2H-pyrimido[6,1-a]isoquinolin-3(4H)-yl)ethyl)-4-hydroxy-1-methyl-1H-1,2,3-triazole-5-carboxamide **(Compound 8)**

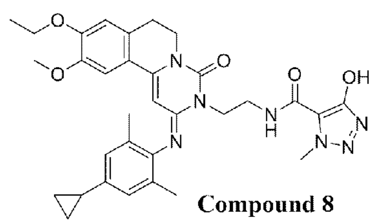

Compound 8

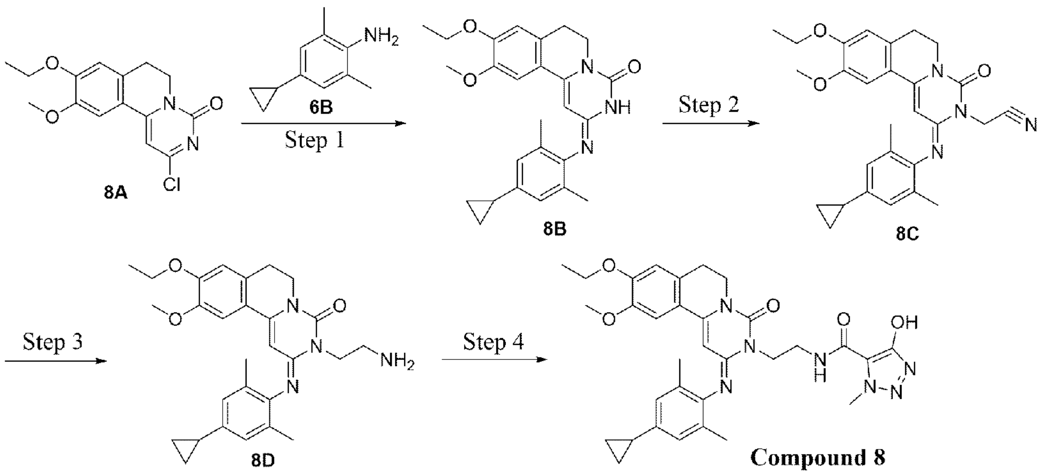


8A 6B 8B 8C 8D Compound 8

Step 1:

Compound **8A** (0.23 g, 0.75 mmol) (prepared with reference to WO 2020011254) was dissolved in isopropanol (12 mL), **6B** (0.18 g, 1.12 mmol) was added, and the mixture was reacted at 90°C for 24 hours after the addition was completed. After the reaction was completed, the reaction solution was cooled to room temperature and concentrated under reduced pressure, saturated sodium bicarbonate aqueous solution (20 mL) was added, and the mixture was extracted with dichloromethane (20 mL × 3). The organic phases were combined, washed with saturated sodium chloride aqueous solution (20 mL), dried over anhydrous sodium sulfate and filtered, the filtrate was concentrated under reduced pressure, and the residue was separated by silica gel column chromatography (DCM : MeOH (v/v) = 20 : 1), to afford compound **8B** (0.28 g, yield: 87%).

LC-MS (ESI): m/z =432.2 $[M+H]^+$.

Step 2:

**Compound 8B** (0.28 g, 0.65 mmol), bromoacetonitrile (0.24 g, 1.95 mmol) and lithium carbonate (0.15 g, 1.95 mmol) were successively dissolved in acetonitrile (20 mL), and the mixture was reacted at 90°C for 16 hours. After the reaction was completed, the reaction solution was cooled to room temperature and filtered, the filter cake was washed with dichloromethane (20 mL), and the filtrate was concentrated under reduced pressure and then separated by silica gel column chromatography (DCM : MeOH (v/v) = 10 : 1), to afford the target compound **8C** (0.22 g, yield: 72%).

LC-MS (ESI): m/z =471.3 $[M+H]^+$.

Step 3:

Compound **8C** (0.22 g, 0.47 mmol) was dissolved in 7 N ammonia in methanol (10 mL), Raney nickel (0.5 g, 50 wt%) was added, and the mixture was reacted under hydrogen atmosphere of 10 bar for 16 hours. After the reaction was completed, the reaction solution was filtered, the filtrate was concentrated under reduced pressure, and the residue was separated by silica gel column chromatography (dichloromethane: methanol (v/v) = 10 : 1-5 : 1), to afford compound **8D** (0.18 g, yield: 81%).

LC-MS (ESI): m/z = 475.2 $[M+H]^+$.

Step 4:

4-hydroxy-1-methyl-1H-1,2,3-triazole-5-carboxylic acid **(1H)** (81.5 mg, 0.57 mmol) was dissolved in DMF (15 mL), DIPEA (220 mg, 1.71 mmol) and HATU (216.6 mg, 0.57 mmol) were added at room temperature, followed by stirring for 10 minutes, then compound **8D** (180 mg, 0.38 mmol) was added, and the mixture was reacted at room temperature for 3 hours. After the reaction was completed as monitored by TLC/LCMS, the reaction was quenched with saturated sodium chloride aqueous solution (20 mL), and the mixture was extracted with dichloromethane (30 mL × 3). The organic phases were combined, dried over anhydrous sodium sulfate, filtered and concentrated under reduced pressure, and the resulting residue was purified by silica gel column chromatography (dichloromethane : methanol = 20 : 1), to afford the **compound 8** (40 mg, yield: 18%).

LC-MS (ESI): m/z = 600.3 $[M+H]^+$.

$^1$H NMR (400 MHz, Chloroform-d) δ 6.76 (s, 2H), 6.71 (s, 1H), 6.64 (s, 1H), 5.46 (s, 1H), 4.61 (m, 2H), 4.24 (s, 3H),

4.13-4.10 (m, 2H), 4.08-4.04 (m, 2H), 3.92-3.90 (m, 2H), 3.75 (s, 3H), 2.89-2.86 (m, 2H), 2.04 (s, 6H), 1.49-1.45 (m, 3H), 1.27-1.25 (m, 2H), 0.91-0.86 (m, 2H), 0.64-0.60 (m, 2H).

**Example 9:** (*E*)-N-(2-(9-ethoxy-10-methoxy-2-((4-methoxy-2,6-dimethylphenyl)imino)-4-oxo-6,7-dihydro-2H-pyrimido [6,1-a]isoquinolin-3 (4H)-yl)ethyl)-4-hydroxy-1-methyl-1H-1,2,3-triazole-5-carboxamide **(Compound 9)**

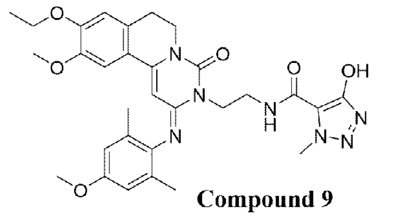

**Compound 9**

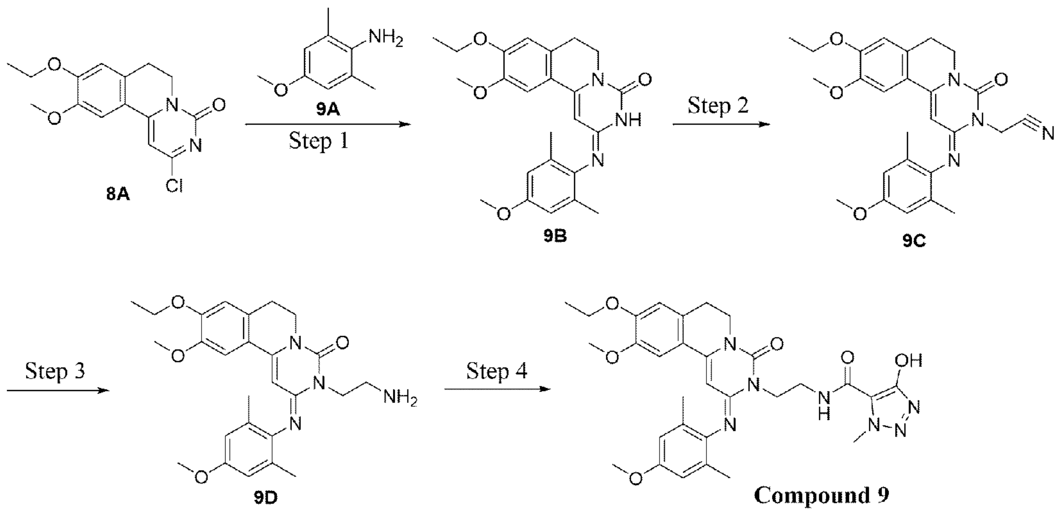


8A 9A 9B 9C 9D Compound 9

Step 1:

Compound **8A** (0.70 g, 2.28 mmol) (prepared with reference to WO 2020011254) was dissolved in isopropanol (20 mL), 4-methoxy-2,6-dimethylaniline (0.34 g, 2.28 mmol) was added, and the mixture was reacted at 90°C for 24 hours after the addition was completed. After the reaction was completed, the mixture was cooled to room temperature. The reaction solution was concentrated under reduced pressure to remove most of solvents, saturated sodium bicarbonate aqueous solution (20 mL) was added to the residue, and the mixture was extracted with dichloromethane (20 mL × 3). The organic phases were combined, washed with saturated sodium chloride aqueous solution (20 mL), dried over anhydrous sodium sulfate, filtered and concentrated, and the resulting crude product was separated by silica gel column chromatography (DCM : MeOH (v/v) = 20 : 1), to afford compound **9B** (0.80 g, yield: 83%).

LC-MS (ESI): m/z =422.3 $[M+H]^+$.

Step 2:

**Compound 9B** (0.80 g, 1.90 mmol), bromoacetonitrile (0.69 g, 5.74 mmol) and lithium carbonate (0.44 g, 5.74 mmol) were successively dissolved in acetonitrile (50 mL), and the mixture was reacted at 90°C for 16 hours. After the reaction was completed, the reaction solution was cooled to room temperature and filtered, the filter cake was washed with dichloromethane (20 mL), the filtrate was concentrated under reduced pressure, and the resulting crude product was separated by silica gel column chromatography (DCM : MeOH (v/v) = 10 : 1), to afford the target compound **9C** (0.60 g, yield: 68%).

LC-MS (ESI): m/z =461.3 $[M+H]^+$.

Step 3:

Compound **9C** (0.60 g, 1.30 mmol) was dissolved in 7 N ammonia in methanol (20 mL), Raney nickel (1.2 g, 50 wt%) was added, and the mixture was reacted under hydrogen atmosphere of 10 bar for 16 hours. After the reaction was completed, the reaction solution was filtered, the filtrate was concentrated under reduced pressure, and the resulting crude product was separated by silica gel column chromatography (dichloromethane: methanol (v/v) = 10 : 1-5 : 1), to afford compound **9D** (0.50 g, yield: 82%).
LC-MS (ESI): m/z = 465.2 $[M+H]^+$.

Step 4:

4-hydroxy-1-methyl-1H-1,2,3-triazole-5-carboxylic acid **(1H)** (92 mg, 0.65 mmol) was dissolved in DMF (15 mL), DIPEA (250 mg, 1.94 mmol) and HATU (250 mg, 0.65 mmol) were successively added at room temperature, followed by stirring for 10 minutes, then compound **9D** (200 mg, 0.43 mmol) was added, and the mixture was reacted at room temperature for 3 hours. After the reaction was completed as monitored by TLC/LCMS, the reaction was quenched with saturated sodium chloride aqueous solution (20 mL), and the mixture was extracted with dichloromethane (30 mL × 3). The organic phases were combined, dried over anhydrous sodium sulfate, filtered and concentrated under reduced pressure, and the resulting residue was purified by silica gel column chromatography (dichloromethane : methanol = 20 : 1), to afford the **compound 9** (60 mg, yield: 23%).
LC-MS (ESI): m/z = 590.3 $[M+H]^+$.
$^1$H NMR (400 MHz, Chloroform-d) δ 6.73 (s, 1H), 6.64 (s, 1H), 6.62 (s, 2H), 5.47 (s, 1H), 4.58-4.55 (m, 2H), 4.26 (s, 3H), 4.13-4.03 (m, 5H), 3.92-3.90 (m, 2H), 3.77 (s, 3H), 3.75 (s, 3H), 2.88-2.85 (m, 2H), 2.05 (s, 6H), 1.48-1.45 (m, 3H).

**Example 10:** (*E*)-N-(2-(2-((3,5-dimethylphenyl)imino)-9-ethoxy-10-methoxy-4-oxo-6,7-dihydro-2H-pyrimido[6,1-a]isoquinolin-3(4H)-yl)ethyl)-4-hydroxy-1-methyl-1H-1,2,3-triazole-5-carboxamide **(Compound 10)**

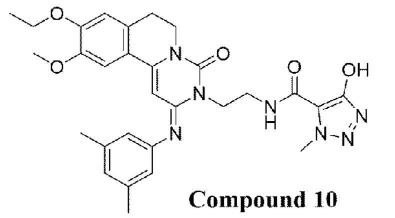

**Compound 10**

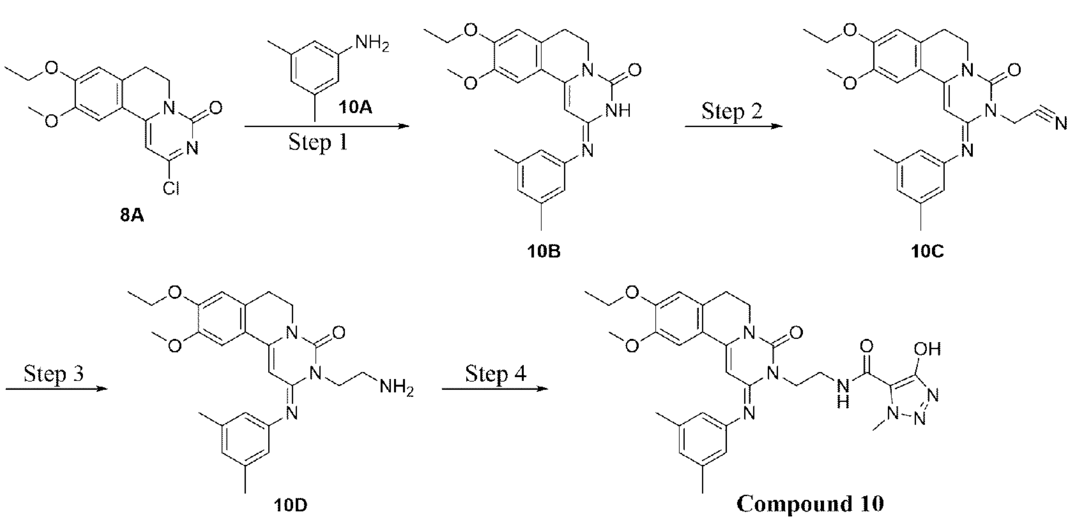


8A, 10A, 10B, 10C, 10D, **Compound 10**

Step 1:

Compound **8A** (0.20 g, 0.65 mmol) (prepared with reference to WO 2020011254) was dissolved in isopropanol (20 mL), 1-amino-3,5-dimethylaniline (0.08 g, 0.65 mmol) was added, and the mixture was reacted at 90°C for 24 hours

after the addition was completed. After the addition was completed, the reaction solution was cooled to room temperature and concentrated under reduced pressure to remove most of reaction solvents, saturated sodium bicarbonate aqueous solution (20 mL) was added to the residue, and the mixture was extracted with dichloromethane (20 mL × 3). The organic phases were combined, washed with saturated sodium chloride aqueous solution (20 mL), dried over anhydrous sodium sulfate and filtered, the filtrate was concentrated under reduced pressure, and the resulting crude product was separated by silica gel column chromatography (DCM : MeOH (v/v) = 20 : 1), to afford compound **10B** (0.20 g, yield: 80%).

LC-MS (ESI): m/z =392.3 $[M+H]^+$.

Step 2:

**Compound 10B** (0.20 g, 0.51 mmol), bromoacetonitrile (0.18 g, 1.53 mmol) and lithium carbonate (0.11 g, 1.53 mmol) were successively added to acetonitrile (20 mL), and the mixture was reacted at 90°C for 16 hours. After the reaction was completed, the reaction solution was cooled to room temperature and filtered, the filter cake was washed with dichloromethane (20 mL), the filtrate was concentrated under reduced pressure and then separated by silica gel column chromatography (DCM : MeOH (v/v) = 10 : 1), to afford the target compound **10C** (0.15 g, yield: 68%).

LC-MS (ESI): m/z =431.3 $[M+H]^+$.

Step 3:

Compound **10C** (0.15 g, 0.35 mmol) was dissolved in 7 N ammonia in methanol (10 mL), Raney nickel (0.2 g, 50 wt%) was added, and the mixture was reacted under hydrogen atmosphere of 10 bar for 16 hours. After the reaction was completed, the reaction solution was filtered, the filtrate was concentrated under reduced pressure, and the residue was separated by silica gel column chromatography (dichloromethane : methanol (v/v) = 10 : 1-5 : 1), to afford compound **10D** (65 mg, yield: 43%).

LC-MS (ESI): m/z = 435.2 $[M+H]^+$.

Step 4:

4-hydroxy-1-methyl-1H-1,2,3-triazole-5-carboxylic acid **(1H)** (32 mg, 0.22 mmol) was dissolved in DMF (10 mL), DIPEA (58 mg, 0.45 mmol) and HATU (84 mg, 0.22 mmol) were successively added at room temperature, followed by stirring for 10 minutes, then compound **10D** (65 mg, 0.15 mmol) was added, and the mixture was reacted at room temperature for 3 hours. After the reaction was completed as monitored by TLC/LCMS, the reaction was quenched with saturated sodium chloride aqueous solution (20 mL), and the mixture was extracted with dichloromethane (30 mL × 3). The organic phases were combined, dried over anhydrous sodium sulfate, filtered and concentrated under reduced pressure, and the resulting residue was purified by silica gel column chromatography (dichloromethane : methanol = 20 : 1), to afford the **compound 10** (20 mg, yield: 24%).

LC-MS (ESI): m/z = 560.3 $[M+H]^+$.

$^1H$ NMR (400 MHz, Chloroform-d) δ 6.80 (s, 1H), 6.64 (s, 1H), 6.62 (s, 1H), 6.44 (s, 2H), 5.96 (s, 1H), 4.51-4.48 (m, 2H), 4.21 (s, 3H), 4.13-4.02 (m, 5H), 3.85-3.80 (m, 2H), 3.76 (s, 3H), 2.87-2.84 (m, 2H), 2.22 (s, 6H), 1.49-1.45 (m, 3H).

**Example 11:** (*E*)-N-(2-(9-ethoxy-2-(mesitylimino)-4-oxo-6,7-dihydro-2H-pyrimido[6,1 -a]isoquinolin-3 (4H)-yl)ethyl)-4-hydroxy-1 -methyl- 1H-1,2,3 -triazole-5-carboxamide **(Compound 11)**

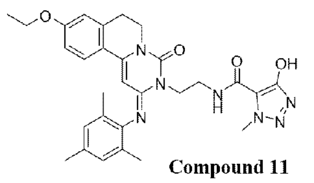

Compound 11

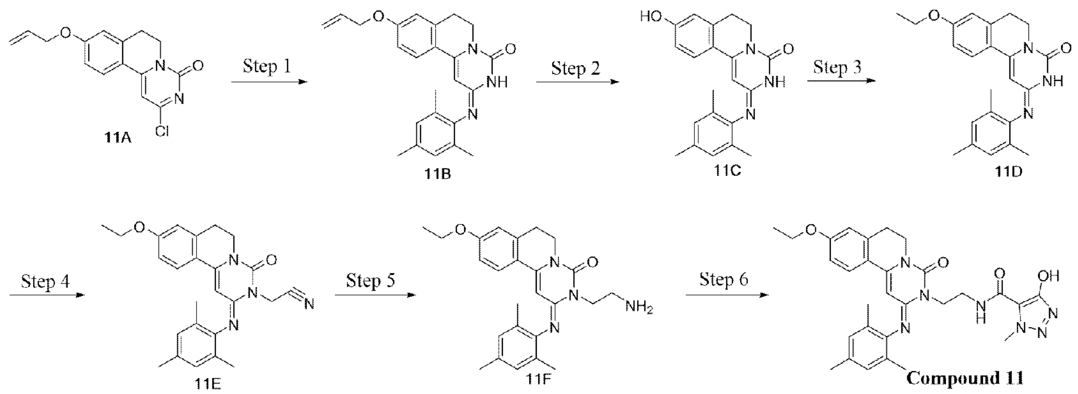


11A 11B 11C 11D 11E 11F Compound 11

Step 1:

Compound **11A** (5.1 g, 17.7 mmol) (prepared with reference to WO 2014095798) was dissolved in isopropanol (100 mL), 2,4,6- trimethyl aniline (4.78 g, 35.3 mmol) was added, and the mixture was reacted at 90°C for 24 hours after the addition was completed. After the reaction was completed, the reaction solution was cooled to room temperature and concentrated under reduced pressure to remove most of reaction solvents, saturated sodium bicarbonate aqueous solution (200 mL) was added to the residue, and the mixture was extracted with dichloromethane (200 mL × 3). The organic phases were combined, washed with saturated sodium chloride aqueous solution (200 mL), dried over anhydrous sodium sulfate and filtered, the filtrate was concentrated under reduced pressure, and the residue was separated by silica gel column chromatography (DCM : MeOH (v/v) = 20 : 1), to afford compound **11B** (5.3 g, yield: 77%).

LC-MS (ESI): m/z =388.2 $[M+H]^+$.

Step 2:

**Compound 11B** (0.39 g, 1.0 mmol), tetrakistriphenylphosphine palladium (0.12 g, 0.1 mmol), and potassium carbonate (0.28 g, 2 mmol) were successively added to dichloromethane (10 mL) and methanol (10 mL), and the mixture was stirred at room temperature for 2 hours. After the reaction was completed, the reaction solution was filtered, the filter cake was washed with methanol (20 mL) and the filtrate was concentrated under reduced pressure to afford a crude product which was directly used for the next step.

Step 3:

Compound **11C** (0.32 g, 0.92 mmol) was dissolved in DMF (10 mL), potassium carbonate (0.28 g, 2 mmol) was added, the mixture was stirred until uniform, then iodoethane (0.22 g, 1.4 mmol) was added, and the mixture was reacted at room temperature for 30 minutes. After the reaction was completed, the reaction solution was filtered, the filtrate was concentrated under reduced pressure, and the residue was separated by silica gel column chromatography (dichloromethane : methanol (v/v) = 20 : 1), to afford compound **11D** (0.31 g, yield: 93%).

LC-MS (ESI): m/z =376.3 $[M+H]^+$.

Step 4:

**Compound 11D** (0.31 g, 0.83 mmol), bromoacetonitrile (0.24 g, 2 mmol) and lithium carbonate (0.15 g, 2 mmol) were successively dissolved in acetonitrile (10 mL), and the mixture was reacted at 80°C for 16 hours. After the reaction was completed, the reaction solution was cooled to room temperature and filtered, the filter cake was washed with methanol (20 mL), and the filtrate was concentrated under reduced pressure and then separated by silica gel column chromatography (DCM : MeOH (v/v) = 10 : 1), to afford the target compound **11E** (0.24 g, yield: 70%).

LC-MS (ESI): m/z =415.3 $[M+H]^+$.

Step 5:

Compound **11E** (0.24 g, 0.58 mmol) was dissolved in 7 N ammonia in methanol (15 mL), Raney nickel (0.6 g, 50 wt%) was added, and the mixture was reacted at room temperature under hydrogen atmosphere of 10 bar for 16 hours. After the reaction was completed, the reaction solution was filtered, the filtrate was concentrated under reduced pressure, and the residue was separated by silica gel column chromatography (dichloromethane : methanol (v/v) = 10 : 1-5 : 1), to

afford compound **11F** (0.14 g, yield: 58%).

LC-MS (ESI): m/z = 419.3 $[M+H]^+$.

Step 6:

4-hydroxy-1-methyl-1H-1,2,3-triazole-5-carboxylic acid **(1H)** (71 mg, 0.5 mmol) was dissolved in DMF (5 mL), DIPEA (0.13 g, 1 mmol) and HATU (0.19 g, 0.5 mmol) were successively added at room temperature, followed by stirring for 10 minutes, then compound **11F** (0.14 g, 0.33 mmol) was added, and the mixture was reacted at room temperature for 1 hour. The reaction was quenched with saturated sodium chloride aqueous solution (20 mL), and the mixture was extracted with dichloromethane (30 mL × 2). The organic phases were combined, dried over anhydrous sodium sulfate, filtered and concentrated under reduced pressure, and the resulting residue was purified by silica gel column chromatography (dichloromethane : methanol = 20 : 1), to afford the **compound 11** (0.12 g, yield: 66%).

LC-MS (ESI): m/z = 544.3 $[M+H]^+$.

$^1$H NMR (400 MHz, Chloroform-*d*) δ 7.24 (s, 1H), 6.88 (s, 2H), 6.78 - 6.73 (m, 1H), 6.71 (s, 1H), 5.55 (s, 1H), 4.72 (s, 2H), 4.21 (s, 3H), 4.12 (t, 2H), 4.05 (q, 2H), 3.96 (s, 2H), 2.97 (t, 2H), 2.29 (s, 3H), 2.06 (s, 6H), 1.42 (t, 3H).

Biological test:

1. In vitro assay of the inhibitory activity of compounds against PDE 3A and PDE 4B2 enzymes

**Experimental materials:**

Envision 2104 Multilabel Reader (PerkinElmer)
Black 96-well Plate (Cat#6005540, PerkinElmer)
PDE3A Assay Kit (BPS, Cat. 79736), PDE4B2 Assay Kit (BPS, Cat. 60343)

- PDE3A recombinant enzyme
- PDE4B2 recombinant enzyme
- FAM-Cyclic-3', 5'-AMP
- PDE assay buffer
- Binding Agent

Binding Agent Diluent (cAMP)

**Experimental procedure:**

1) Prepare FAM cAMP working solution: 20 μL of FAM-Cyclic-3', 5'-AMP stock was added to 1980 μL of PDE assay buffer. The mixture was added to all wells at 25 μL/well.
2) Prepare compound solution: the test compounds were dissolved in DMSO to give a 10 mM stock solution. The compound stock solution was diluted with DMSO to a 100 X Top Dose diluent. 5 μL of 100 X Top Dose diluent was added to 45 μL of PDE assay buffer to prepare a compound Top Dose working solution. Then, the compound working solution was subjected to double dilution with PDE assay buffer containing 10% DMSO to prepare compound working solutions of various concentrations. The compound working solutions were added to compound wells at 5 μL/well. 5 μL of PDE assay buffer containing 10% DMSO was added to each control well.
3) Prepare PDE enzyme solution: PDE3A and PDE4B2 enzyme stock solutions were diluted to 150 pg/μL and 50 pg/μL, respectively, with PDE assay buffer, and added to all compound wells and Vehicle control wells at 20 μL/well. 20 μL of PDE assay buffer was added to Blank control wells.
4) React at room temperature for 1 h.
5) Prepare Binding Agent: 80 μL of binding agent was added to 7920 μL of binding agent diluent, and the mixture was mixed well and added to all wells at 100 μL/well.
6) React at room temperature for 1 h.
7) Read FP on Envision.

8) Calculation formula for original data:

$$\textit{\%Inhibition rate} = (FP_V - FP_S)/(FP_V - FP_B) \times 100\%$$

$FP_S$ = sample FP
$FP_V$ = Vehicle control FP
$FP_B$ = Blank control FP.
Nonlinear regression analysis of log[dose]-inhibition rate was performed by graphpad and $IC_{50}$ was calculated.

**Experimental results:**

The compounds of the present invention had excellent PDE 3 and PDE 4 inhibitory activity, with $IC_{50}$ values below 500 nM. Some compounds with good inhibitory activity had $IC_{50}$ < 200 nM, some compounds with better inhibitory activity had $IC_{50}$ < 100 nM, and some compounds with excellent inhibitory activity had $IC_{50}$ < 50 nM. The test results of some compounds are listed in the table below.

Table 1 Results of in vitro screening assay of compounds

| Compound | PDE3A $IC_{50}$ (nm) | PDE 4B2 $IC_{50}$ (nm) |
|---|---|---|
| 6 | 0.53 | 8.43 |
| 8 | 0.59 | 1.40 |
| 9 | 0.50 | 1.52 |
| Compound A | 0.48 | 11.04 |

Compound A is the compound WX036 in patent WO 2020011254, which is synthesized with reference to the method described in this patent.
Conclusion: The compound of the present invention has good inhibitory activity against PDE 3 and PDE 4, especially compound 8.

## Claims

**1.** A compound of formula (I), or a stereoisomer, solvate, or pharmaceutically acceptable salt thereof,

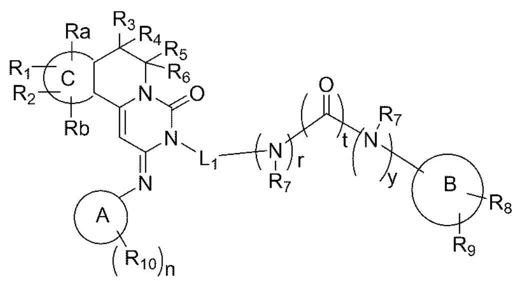

I

**characterized in that** ring A is phenyl, a $C_{9-10}$ fused carbocycle or 5- to 6-membered heteroaryl containing 1-3 heteroatoms selected from N, S and O;
ring B is a 4- to 10-membered heterocycle containing 1-3 heteroatoms selected from N, S and O or a $C_{3-10}$ carbocycle;
ring C is phenyl or a 5- to 6-membered heteroaryl containing 1-3 heteroatoms selected from N, S and O;
$R_1$, $R_2$, $R_a$ and $R_b$ are independently H, deuterium, halogen, CN, OH, $NH_2$, - $NHC_{1-4}$alkyl, -$N(C_{1-4}alkyl)_2$, -$OC_{1-4}$alkyl, -$O(CH_2)_mC_{3-6}$cycloalkyl, - $O(CH_2)_m$phenyl, -$O(CH_2)_m$-4- to 7-membered heterocycloalkyl containing 1-3 heteroatoms selected from N, S and O, -$O(CH_2)_m$-5- to 6-membered heteroaryl containing 1-3 heteroatoms selected from N, S and O, $C_{1-4}$alkyl, $C_{2-4}$alkenyl, $C_{2-4}$alkynyl, -$(CH_2)_mC_{3-6}$cycloalkyl, -$(CH_2)_m$phenyl,

$-(CH_2)_m$-4- to 7-membered heterocycloalkyl containing 1-3 heteroatoms selected from N, S and O, and -$(CH_2)_m$-5- to 6-membered heteroaryl containing 1-3 heteroatoms selected from N, S and O or are not present, wherein the alkyl, phenyl, cycloalkyl, heterocycloalkyl and heteroaryl are optionally further substituted with 1-3 groups selected from deuterium, halogen, $C_{1-4}$alkyl, $C_{2-4}$alkenyl, $C_{2-4}$alkynyl, $C_{1-4}$alkoxy, deuterated $C_{1-4}$alkyl, deuterated $C_{1-4}$alkoxy, halogenated $C_{1-4}$alkoxy, CN, $NH_2$, -NH$C_{1-4}$alkyl, - N($C_{1-4}$alkyl)$_2$ and OH;

optionally, $R_1$ and $R_2$ together with the atom to which they are attached form a 5- to 7-membered heterocycle containing 1-3 heteroatoms selected from N, S and O, or a $C_{4-7}$ carbocycle, wherein the heterocycle and carbocycle are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy;

$R_3$, $R_4$, $R_5$ and $R_6$ are independently H, deuterium, halogen, $C_{1-4}$alkyl, - $(CH_2)_m C_{3-6}$cycloalkyl, wherein the alkyl and cycloalkyl are optionally substituted with 1-3 groups selected from deuterium, halogen, CN and $NH_2$;

optionally, $R_3$ and $R_4$, or $R_5$ and $R_6$ together with the carbon atom to which they are attached form $C_{3-6}$cycloalkyl;

$L_1$ is $C_{1-6}$alkylene, wherein the alkylene is optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy;

each $R_7$ is independently H, $C_{1-4}$alkyl or -$(CH_2)_m C_{3-6}$cycloalkyl, wherein the alkyl and cycloalkyl are optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy;

$R_8$ and $R_9$ are independently H, =O, $C_{1-4}$alkyl, OH, -O$C_{1-4}$alkyl, -NH$C_{1-4}$alkyl, -N($C_{1-4}$alkyl)$_2$, -C(O)$C_{1-4}$alkyl, -C(O)$NH_2$, -C(O)NH$C_{1-4}$alkyl, -C(O)N($C_{1-4}$alkyl)$_2$, CN, halogen and $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy;

each $R_{10}$ is independently H, halogen, CN, $NH_2$, -NH$C_{1-4}$alkyl, -N($C_{1-4}$alkyl)$_2$, OH, $C_{1-4}$alkyl, $C_{2-4}$alkenyl, $C_{2-4}$alkynyl, -$(CH_2)_m$-$C_{3-6}$cycloalkyl, -O-$C_{3-6}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl, $C_{2-4}$alkenyl, $C_{2-4}$alkynyl, CN, $NH_2$, -NH$C_{1-4}$alkyl, -N($C_{1-4}$alkyl)$_2$ and OH;

n is 0, 1, 2, 3 or 4;

each m is independently 0, 1, 2, 3 or 4; and

r, t and y are independently 0 or 1.

2. The compound, or the stereoisomer, solvate, or pharmaceutically acceptable salt thereof according to claim 1, **characterized in that** the compound has a structure of formula II:

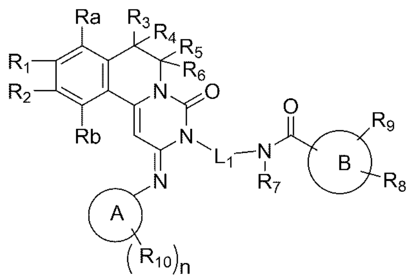

II

wherein

(1) ring B is: phenyl, $C_{3-6}$cycloalkyl, 6-membered heteroaryl containing 1-3 heteroatoms selected from N, S and O, 4- to 7-membered heterocycloalkyl containing 1-3 heteroatoms selected from N, S and O, or 8- to 10-membered fused heterocycle or spirocyclic heterocycle containing 1-3 heteroatoms selected from N, S and O; and

$R_8$ and $R_9$ are independently H, =O, $C_{1-4}$alkyl, OH, -NH$C_{1-4}$alkyl, -N($C_{1-4}$alkyl)$_2$, -C(O)$C_{1-4}$alkyl, -C(O)$NH_2$, -C(O)NH$C_{1-4}$alkyl, -C(O)N($C_{1-4}$alkyl)$_2$, CN, halogen and $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy; or

(2) ring B is:

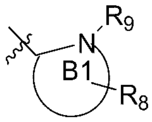,

and $B_1$ is a 5-membered heteroaryl containing 1-3 heteroatoms selected from N, S and O;
$R_8$ is H, $C_{1-4}$alkyl, OH, CN, halogen or $C_{3-6}$cycloalkyl, wherein the alkyl and cycloalkyl are optionally substituted with 1-3 groups selected from deuterium, halogen, $C_{1-4}$alkyl, halogenated $C_{1-4}$alkyl, $C_{2-4}$alkenyl, $C_{2-4}$alkynyl, $C_{1-4}$alkoxy, deuterated $C_{1-4}$alkoxy and halogenated $C_{1-4}$alkoxy; and
$R_9$ is $C_{1-4}$alkyl or $C_{3-6}$cycloalkyl, wherein the alkyl and cycloalkyl are optionally substituted with 1-3 groups selected from deuterium, halogen, $C_{2-4}$alkenyl, $C_{2-4}$alkynyl, $C_{1-4}$alkoxy, deuterated $C_{1-4}$alkoxy and halogenated $C_{1-4}$alkoxy;
provided that, the compound is not:

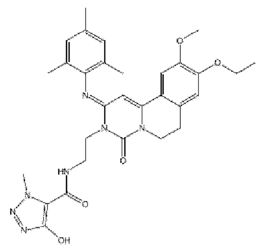

**3.** The compound, or the stereoisomer, solvate, or pharmaceutically acceptable salt thereof according to claim 2,

**characterized in that** ring A is phenyl,

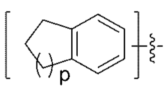,

pyridyl, pyrazolyl, pyrimidinyl, thienyl, thiazolyl, isothiazolyl, oxazolyl or isoxazolyl;
$R_1$, $R_2$, $R_a$ and $R_b$ are independently H, deuterium, halogen, CN, OH, $NH_2$, - $NHC_{1-4}$alkyl, $-N(C_{1-4}alkyl)_2$, $-OC_{1-4}$alkyl, $-O(CH_2)_mC_{3-6}$cycloalkyl, - $O(CH_2)_m$phenyl, $-O(CH_2)_m$-4- to 7-membered heterocycloalkyl containing 1-3 heteroatoms selected from N, S and O, $C_{1-4}$alkyl, $-(CH_2)_mC_{3-6}$cycloalkyl, or - $(CH_2)_m$-4- to 7-membered heterocycloalkyl containing 1-3 heteroatoms selected from N, S and O, wherein the alkyl, cycloalkyl and heterocycloalkyl are optionally further substituted with 1-3 groups selected from deuterium, halogen, $C_{1-4}$alkyl, $C_{2-4}$alkenyl, $C_{2-4}$alkynyl, $C_{1-4}$alkoxy, deuterated $C_{1-4}$alkyl, deuterated $C_{1-4}$alkoxy and halogenated $C_{1-4}$alkoxy;
optionally, $R_1$ and $R_2$ together with the atom to which they are attached form a 5- to 6-membered heterocycle containing 1-3 heteroatoms selected from N, S and O, or a $C_{5-6}$ carbocycle, wherein the heterocycle and carbocycle are optionally substituted with 1-3 groups selected from deuterium, halogen, $C_{1-4}$alkyl, halogenated $C_{1-4}$alkyl, $C_{1-4}$alkoxy, deuterated $C_{1-4}$alkoxy and halogenated $C_{1-4}$alkoxy;
$R_3$, $R_4$, $R_5$ and $R_6$ are independently H, deuterium, halogen and $C_{1-4}$alkyl, wherein the alkyl is optionally substituted with 1-3 groups selected from deuterium, halogen, CN and $NH_2$;
$L_1$ is $C_{2-4}$alkylene, wherein the alkylene is optionally substituted with 1-3 groups selected from deuterium, halogen, $C_{1-4}$alkoxy, deuterated $C_{1-4}$alkoxy and halogenated $C_{1-4}$alkoxy;
$R_7$ is H or $C_{1-4}$alkyl, wherein the alkyl is optionally substituted with 1-3 groups selected from deuterium, halogen, $C_{1-4}$alkoxy, deuterated $C_{1-4}$ alkoxy and halogenated $C_{1-4}$alkoxy;
each $R_{10}$ is independently H, halogen, CN, $C_{1-4}$alkyl, $C_{2-4}$alkenyl, $C_{2-4}$alkynyl, $C_{3-6}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl and CN;
n is 1, 2 or 3;
each m is independently 0, 1, 2, or 3;
p is 1 or 2;
and

(1) ring B is phenyl, $C_{3-6}$cycloalkyl, pyridyl, pyridazinyl, pyrazinyl, a 4- to 6-membered heterocycloalkyl

containing 1-3 heteroatoms selected from N, S and O, or 8- to 10-membered fused heterocycle or spirocyclic heterocycle containing 1-3 heteroatoms selected from N, S and O; and
$R_8$ and $R_9$ are independently H, =O, $C_{1-4}$alkyl, OH, -NH$C_{1-4}$alkyl, -N($C_{1-4}$alkyl)$_2$, -C(O)$C_{1-4}$alkyl, -C(O)$NH_2$, -C(O)NH$C_{1-4}$alkyl, -C(O)N($C_{1-4}$alkyl)$_2$, CN, halogen and $C_{3-6}$cycloalkyl, wherein the alkyl and cycloalkyl are optionally substituted with 1-3 groups selected from deuterium, halogen, $C_{1-4}$alkyl and halogenated $C_{1-4}$alkyl; or
(2) ring B is

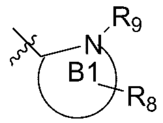

,

and $B_1$ is triazazolyl, oxadiazolyl, imidazolyl or pyrrolyl;

$R_8$ is H, OH, $C_{1-4}$alkyl and $C_{3-6}$cycloalkyl, wherein the alkyl and cycloalkyl are optionally substituted with 1-3 groups selected from deuterium, halogen, $C_{1-4}$alkoxy, deuterated $C_{1-4}$alkoxy and halogenated $C_{1-4}$alkoxy; and
$R_9$ is $C_{1-4}$alkyl or $C_{3-6}$cycloalkyl, wherein the alkyl and cycloalkyl are optionally substituted with 1-3 groups selected from deuterium, halogen, $C_{1-4}$alkoxy, deuterated $C_{1-4}$alkoxy and halogenated $C_{1-4}$alkoxy.

**4.** The compound, or the stereoisomer, solvate, or pharmaceutically acceptable salt thereof according to claim 2, **characterized in that** the compound has a structure of formula III:

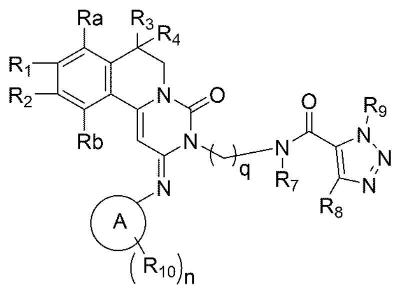

III

wherein $R_1$, $R_2$, $R_a$ and $R_b$ are independently H, deuterium, halogen, CN, OH, -O$C_{1-4}$alkyl, -O($CH_2$)$_m$$C_{3-6}$cycloalkyl, -O($CH_2$)$_m$phenyl, $C_{1-4}$alkyl or -($CH_2$)$_m$$C_{3-6}$cycloalkyl, wherein the alkyl and cycloalkyl are optionally further substituted with 1-3 groups selected from deuterium, halogen, $C_{1-4}$alkyl, $C_{1-4}$alkoxy, deuterated $C_{1-4}$alkyl, deuterated $C_{1-4}$alkoxy and halogenated $C_{1-4}$alkoxy;
optionally, $R_1$ and $R_2$ together with the atom to which they are attached form a 5- to 6-membered heterocycle containing 1-3 heteroatoms selected from N, S and O, or a $C_{5-6}$ carbocycle, wherein the heterocycle and carbocycle are optionally substituted with 1-3 groups selected from deuterium, halogen, $C_{1-4}$alkyl, halogenated $C_{1-4}$alkyl and $C_{1-4}$alkoxy;
$R_3$ and $R_4$ are independently H, deuterium, halogen and $C_{1-4}$alkyl, wherein the alkyl is optionally substituted with 1-3 groups selected from deuterium, F, Cl, Br and I;
ring A is phenyl,

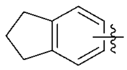

,

thienyl, pyrazolyl, thiazolyl, isothiazolyl, oxazolyl or isoxazolyl;
$R_7$ is H or $C_{1-3}$ alkyl, wherein the alkyl is optionally substituted with 1-3 groups selected from deuterium, halogen, $C_{1-4}$ alkoxy, deuterated $C_{1-4}$ alkoxy and halogenated $C_{1-4}$ alkoxy;
$R_8$ is H, OH or $C_{1-4}$alkyl, wherein the alkyl is optionally substituted with 1-3 groups selected from deuterium, halogen, $C_{1-4}$alkoxy and halogenated $C_{1-4}$alkoxy;
$R_9$ is $C_{1-4}$alkyl or $C_{3-6}$cycloalkyl, wherein the alkyl and cycloalkyl are optionally substituted with 1-3 groups selected from deuterium, halogen, $C_{1-4}$alkoxy and halogenated $C_{1-4}$alkoxy;

each $R_{10}$ is independently H, halogen, CN, $C_{1-4}$alkyl, $C_{2-4}$alkenyl, $C_{2-4}$alkynyl, $C_{3-4}$cycloalkyl or $C_{1-4}$alkoxy, wherein the alkyl, alkenyl, alkynyl, cycloalkyl and alkoxy are optionally substituted with 1-3 groups selected from deuterium, halogen, $C_{1-4}$alkyl and CN;
n is 2 or 3;
each m is independently 0, 1, or 2; and
q is 1, 2 or 3.

**5.** The compound, or the stereoisomer, solvate, or pharmaceutically acceptable salt thereof according to claim 4,

**characterized in that** $R_1$, $R_2$, $R_a$ and $R_b$ are independently H, methoxy, ethoxy, propoxy, isopropoxy, $-O(CH_2)_m$cyclopropyl, benzyloxy, methyl, ethyl, propyl, isopropyl or $-(CH_2)_m$cyclopropyl, wherein the methoxy, ethoxy, propoxy, isopropoxy, benzyloxy, methyl, ethyl, propyl, isopropyl and cyclopropyl are optionally further substituted with 1-3 groups selected from deuterium, F, Cl, Br, I, methyl, ethyl, propyl, isopropyl, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy;
optionally, $R_1$ and $R_2$ together with the atom to which they are attached form

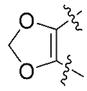
,

and are optionally substituted with 1-2 groups selected from deuterium, F, Cl, Br, I, methyl, ethyl, propyl, methoxy, ethoxy, propoxy, halogenated methyl, halogenated ethyl and halogenated propyl;
$R_3$ and $R_4$ are independently H, deuterium, F, Cl, Br, I, methyl, ethyl or propyl, wherein the methyl, ethyl or propyl is optionally substituted with 1-3 groups selected from deuterium, F, Cl, Br and I;
ring A is phenyl,

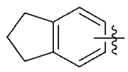
,

thienyl or thiazolyl;
$R_7$ is H, methyl, ethyl, propyl or isopropyl, wherein the methyl, ethyl, propyl or isopropyl is optionally substituted with 1-3 groups selected from deuterium, F, Cl, Br, I, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy;
$R_8$ is H, OH, methyl, ethyl, propyl or isopropyl, wherein the methyl, ethyl, propyl or isopropyl is optionally substituted with 1-3 groups selected from deuterium, halogen, methoxy, ethoxy, propoxy, halogenated methoxy, halogenated ethoxy and halogenated propoxy;
$R_9$ is methyl, ethyl, propyl, isopropyl, cyclopropyl, cyclobutyl or cyclopentyl, wherein the methyl, ethyl, propyl, isopropyl, cyclopropyl, cyclobutyl or cyclopentyl is optionally substituted with 1-3 groups selected from deuterium, halogen, methoxy, ethoxy, propoxy, halogenated methoxy, halogenated ethoxy and halogenated propoxy;
each $R_{10}$ is independently H, F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy, wherein the methyl, ethyl, propyl, isopropyl, n-butyl, isobutyl, ethenyl, propenyl, allyl, ethynyl, propynyl, cyclopropyl, cyclobutyl, methoxy, ethoxy and propoxy are optionally substituted with 1-3 groups selected from deuterium, F, Cl, Br, I, methyl, ethyl, propyl and CN; and
q is 2 or 3.

**6.** The compound, or the stereoisomer, solvate, or pharmaceutically acceptable salt thereof according to claim 2, **characterized in that** the compound has a structure of formula IV:

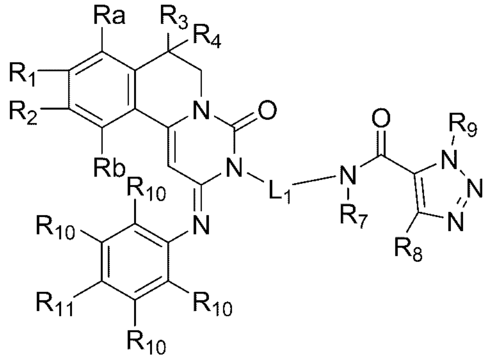

IV

wherein $R_1$, $R_2$, $R_a$ and $R_b$ are independently H, methoxy, ethoxy, propoxy, isopropoxy, -O$(CH_2)_m$cyclopropyl, benzyloxy, methyl, ethyl, propyl, isopropyl or - $(CH_2)_m$cyclopropyl, wherein the methoxy, ethoxy, propoxy, isopropoxy, benzyloxy, methyl, ethyl, propyl, isopropyl and cyclopropyl are optionally further substituted with 1-3 groups selected from deuterium, F, Cl, Br, I, methyl, ethyl, propyl, isopropyl, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy;

$R_3$ and $R_4$ are independently H, deuterium, F, Cl, Br, I, methyl, ethyl or propyl, wherein the methyl, ethyl or propyl is optionally substituted with 1-3 groups selected from deuterium, F, Cl, Br and I;

$L_1$ is $-CH_2CH_2-$ or $-CH_2CH_2CH_2-$;

$R_7$ is H, methyl, ethyl, propyl or isopropyl, wherein the methyl, ethyl, propyl or isopropyl is optionally substituted with 1-3 groups selected from deuterium, F, Cl, Br, I, methoxy, ethoxy, propoxy, deuterated methyl, deuterated ethyl, deuterated propyl, deuterated methoxy, deuterated ethoxy, deuterated propoxy, halogenated methoxy, halogenated ethoxy and halogenated propoxy;

$R_8$ is H, OH, methyl, ethyl, propyl or isopropyl, wherein the methyl, ethyl, propyl or isopropyl is optionally substituted with 1-3 groups selected from deuterium, halogen, methoxy, ethoxy, propoxy, halogenated methoxy, halogenated ethoxy and halogenated propoxy;

$R_9$ is methyl, ethyl, propyl, isopropyl, cyclopropyl, cyclobutyl or cyclopentyl, wherein the methyl, ethyl, propyl, isopropyl, cyclopropyl, cyclobutyl or cyclopentyl is optionally substituted with 1-3 groups selected from deuterium, halogen, methoxy, ethoxy, propoxy, halogenated methoxy, halogenated ethoxy and halogenated propoxy; and

each $R_{10}$ and $R_{11}$ is independently H, F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy, wherein the methyl, ethyl, propyl, isopropyl, n-butyl, isobutyl, ethenyl, propenyl, allyl, ethynyl, propynyl, cyclopropyl, cyclobutyl, methoxy, ethoxy and propoxy are optionally substituted with 1-3 groups selected from deuterium, F, Cl, Br, I, methyl, ethyl, propyl and CN;

provided that when $R_1$ and $R_2$ are both methoxy, $R_{11}$ is F, Cl, Br, I, methyl, ethyl, propyl, isopropyl, n-butyl, isobutyl, ethenyl, propenyl, allyl, ethynyl, propynyl, cyclopropyl, cyclobutyl, methoxy, ethoxy or propoxy.

7. The compound, or the stereoisomer, solvate, or pharmaceutically acceptable salt thereof according to claim 1, **characterized in that** the compound is selected from one of the following structures:

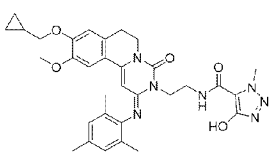
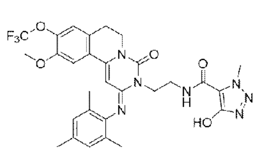
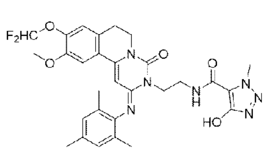
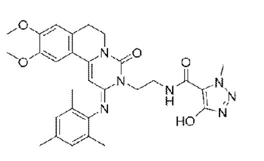

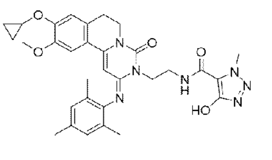
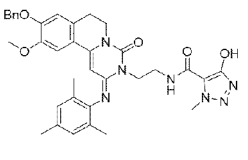
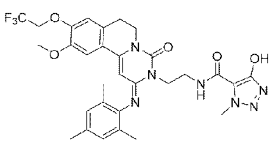
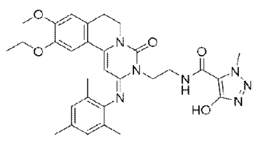

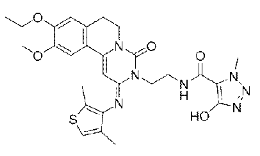 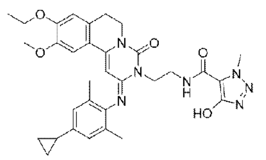 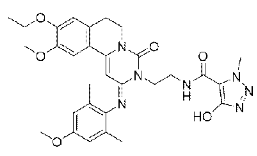 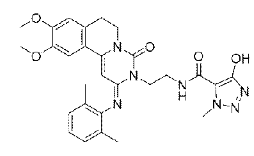

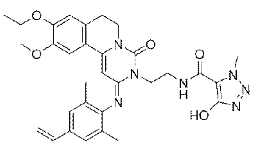 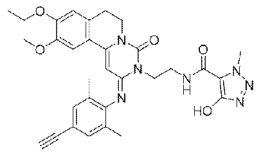 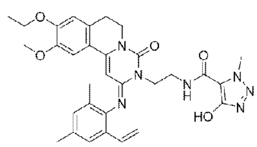 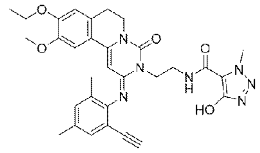

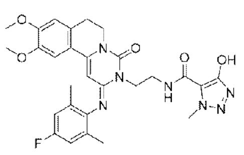 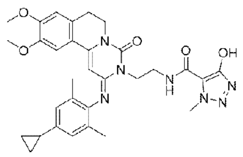 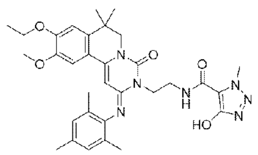 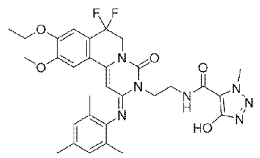

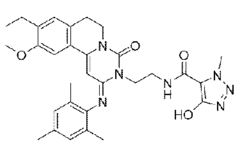 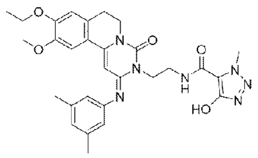 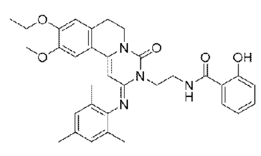 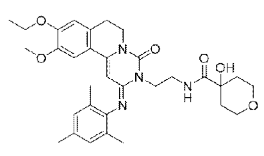

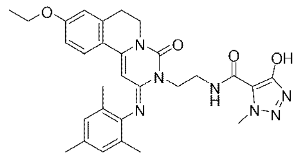

**8.** A pharmaceutical composition comprising the compound, or the stereoisomer, solvate, or pharmaceutically acceptable salt thereof according to any one of claims 1-7, and a pharmaceutically acceptable carrier and/or excipient.

**9.** Use of the compound, or the stereoisomer, solvate, or pharmaceutically acceptable salt thereof according to any one of claims 1-7, or the composition according to claim 8 in the preparation of a drug for the treatment/prevention of PDE3/4-mediated diseases.

**10.** The use according to claim 9, **characterized in that** the PDE3/4-mediated diseases are selected from COPD and asthma.

**11.** A pharmaceutical composition or pharmaceutical preparation comprising 1-1500 mg of the compound, or the stereoisomer, solvate or pharmaceutically acceptable salt thereof according to any one of claims 1-7, and a pharmaceutically acceptable carrier and/or excipient.

**12.** A method for treating a disease in a mammal, **characterized by** administering to a subject a therapeutically effective amount of the compound, or the stereoisomer, solvate, or pharmaceutically acceptable salt thereof according to any one of claims 1-7, wherein the therapeutically effective amount is preferably 1-1500 mg, and the disease is preferably COPD and asthma.

## INTERNATIONAL SEARCH REPORT

International application No.
**PCT/CN2023/073288**

**A. CLASSIFICATION OF SUBJECT MATTER**

C07D471/04(2006.01)i;A61K31/519(2006.01)i;A61K31/517(2006.01)i;A61P11/08(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNTXT; CNABS; DWPI; ENTXT; CJFD; CNKI; STN-REGISTRY; STN-CAPLUS; PATENTICS; 超星读秀, CHAOXING DUXIU; ISI-Web of Science: 海思科, 嘧啶酮, 亚胺, 异喹啉, 三环, 稠合, 酰胺, 脲, PDE?, 慢性阻塞性肺病, 哮喘, +pyrimidinone, +imino+, +isoquinolin+, tricycle, condensed, amide, urea, COPD, chronic obstructive pulmonary disease, asthma

**C. DOCUMENTS CONSIDERED TO BE RELEVANT**

| Category* | Citation of document, with indication, where appropriate, of the relevant passages | Relevant to claim No. |
|---|---|---|
| X | WO 2021143843 A1 (CHIA TAI TIANQING PHARMACEUTICAL GROUP CO., LTD.) 22 July 2021 (2021-07-22)<br>claims 1, 20, 22, 30 | 1, 8-11 |
| X | WO 2020011254 A1 (CHIA TAI TIANQING PHARMACEUTICAL GROUP CO., LTD. et al.) 16 January 2020 (2020-01-16)<br>claims 1, 13, 14, description embodiments 4, 9-60 | 1-11 |
| X | CN 1348453 A (VILNALIS LIMITED) 08 May 2002 (2002-05-08)<br>description embodiments 6, 9, 10-12, figure 1 | 1, 8-11 |
| X | WO 2021143841 A1 (CHIA TAI TIANQING PHARMACEUTICAL GROUP CO., LTD.) 22 July 2021 (2021-07-22)<br>claims 1-19 | 1, 8-11 |
| A | CN 101321758 A (ASTRAZENECA AB) 10 December 2008 (2008-12-10)<br>entire document | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 March 2023** | **29 April 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2023/073288**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:

Claim 12 sets forth a method for treating diseases in a mammal, the method comprising administering to a subject a therapeutically effective amount of the compound of any one of claims 1-7 or a stereoisomer, solvate, or pharmaceutically acceptable salt thereof, wherein the therapeutically effective amount being preferably 1-1500 mg, and the diseases being preferably COPD and asthma. The method belongs to the scope of treatment methods for the human or animal body, that is, belongs to the cases set forth in PCT Rule 39.1(iv) for which an international search is not required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/073288**

| Patent document cited in search report | | | Publication date (day/month/year) | Patent family member(s) | | | Publication date (day/month/year) |
|---|---|---|---|---|---|---|---|
| WO | 2021143843 | A1 | 22 July 2021 | CA | 3167381 | A1 | 22 July 2021 |
| | | | | AU | 2021207159 | A1 | 18 August 2022 |
| | | | | CN | 114929701 | A | 19 August 2022 |
| | | | | EP | 4092022 | A1 | 23 November 2022 |
| WO | 2020011254 | A1 | 16 January 2020 | CA | 3106052 | A1 | 16 January 2020 |
| | | | | IL | 280075 | A | 01 March 2021 |
| | | | | US | 2021323960 | A1 | 21 October 2021 |
| | | | | AU | 2019302610 | A1 | 04 March 2021 |
| | | | | EP | 3822272 | A1 | 19 May 2021 |
| | | | | EP | 3822272 | A4 | 06 April 2022 |
| | | | | KR | 20210031727 | A | 22 March 2021 |
| | | | | JP | 2021532088 | A | 25 November 2021 |
| | | | | CN | 112368281 | A | 12 February 2021 |
| | | | | IN | 202117003115 | A | 26 March 2021 |
| | | | | HK | 40038640 | A0 | 25 June 2021 |
| | | | | CN | 112368281 | B | 18 February 2022 |
| | | | | CN | 114644630 | A | 21 June 2022 |
| | | | | RU | 2021102356 | A | 16 August 2022 |
| CN | 1348453 | A | 08 May 2002 | US | 2004171828 | A1 | 02 September 2004 |
| | | | | US | 7105663 | B2 | 12 September 2006 |
| | | | | US | 2004176353 | A1 | 09 September 2004 |
| | | | | US | 7378424 | B2 | 27 May 2008 |
| | | | | BR | 0009446 | A | 15 January 2002 |
| | | | | BRPI | 0009446 | B1 | 01 November 2016 |
| | | | | US | 2008206163 | A1 | 28 August 2008 |
| | | | | US | 8242127 | B2 | 14 August 2012 |
| | | | | JP | 2002540207 | A | 26 November 2002 |
| | | | | JP | 4685243 | B2 | 18 May 2011 |
| | | | | DE | 60005493 | D1 | 30 October 2003 |
| | | | | WO | 0058308 | A1 | 05 October 2000 |
| | | | | ES | 2208310 | T3 | 16 June 2004 |
| | | | | US | 2012302533 | A1 | 29 November 2012 |
| | | | | AU | 4127400 | A | 16 October 2000 |
| | | | | AU | 773504 | B2 | 27 May 2004 |
| | | | | EP | 1165558 | A1 | 02 January 2002 |
| | | | | EP | 1165558 | B1 | 24 September 2003 |
| | | | | CA | 2368413 | C | 29 July 2008 |
| | | | | US | 2003036542 | A1 | 20 February 2003 |
| | | | | US | 6794391 | B2 | 21 September 2004 |
| | | | | NO | 20014728 | A | 23 November 2001 |
| | | | | NZ | 514158 | A | 29 April 2003 |
| | | | | MX | 2001009846 | A1 | 01 June 2003 |
| | | | | MX | 226070 | B | 03 February 2005 |
| | | | | CN | 100415743 | C | 03 September 2008 |
| WO | 2021143841 | A1 | 22 July 2021 | CA | 3167378 | A1 | 22 July 2021 |
| | | | | AU | 2021208129 | A1 | 18 August 2022 |
| | | | | KR | 20220127845 | A | 20 September 2022 |
| | | | | EP | 4092025 | A1 | 23 November 2022 |
| | | | | CN | 114929700 | A | 19 August 2022 |
| CN | 101321758 | A | 10 December 2008 | WO | 2007040435 | A1 | 12 April 2007 |

Form PCT/ISA/210 (patent family annex) (July 2022)

**INTERNATIONAL SEARCH REPORT**
**Information on patent family members**

International application No.
**PCT/CN2023/073288**

| Patent document cited in search report | Publication date (day/month/year) | Patent family member(s) | | | Publication date (day/month/year) |
|---|---|---|---|---|---|
| | | TW | 200745106 | A | 16 December 2007 |
| | | EP | 1931672 | A1 | 18 June 2008 |
| | | US | 2009054413 | A1 | 26 February 2009 |
| | | JP | 2009510159 | A | 12 March 2009 |
| | | IN | 200803380 | P1 | 20 March 2009 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2000058308 A1
- WO 2020011254 A
- WO 00058308 A
- WO 2014095798 A


### Non-patent literature cited in the description


- Synthetic Organic Chemistry. John Wiley & Sons, Inc
- **S. R. SANDLER et al.** Organic Functional Group Preparations. Academic Press, 1983
- **H. O. HOUSE**. Modern Synthetic Reactions. W. A. Benjamin, Inc, 1972
- **T. L. GILCHRIST**. Heterocyclic Chemistry. John Wiley & Sons, 1992
- **J. MARCH**. Advanced Organic Chemistry: Reactions, Mechanisms and Structure. Wiley-Interscience, 1992
- **FUHRHOP, J.** ; **PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C**. Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modern Carbonyl Chemistry. Wiley-VCH, 2000
- **PATAI, S.** Patai's 1992 Guide to the Chemistry of Functional Groups. Interscience, 1992
- **SOLOMONS, T. W. G.** Organic Chemistry. John Wiley & Sons, 2000
- **STOWELL, J.C**. Intermediate Organic Chemistry. Wiley-Interscience, 1993
- Industrial Organic Chemicals: Starting Materials and Intermediates: An Ullmann's Encyclopedia. John Wiley & Sons, 1999, vol. 8
- Organic Reactions. John Wiley & Sons, 1942, vol. 55
- Chemistry of Functional Groups. John Wiley & Sons, vol. 73
- **P. H. STAHL** ; **C. G. WERMUTH**. Handbook of Pharmaceutical Salts. Verlag Helvetica Chimica Acta, 2002
- *J. Med. Chem.*, 2013, vol. 56 (23), 9673-9682